# EUROPÄISCHE PATENTSCHRIFT

(11) **EP 3 629 889 B1**
(45) Veröffentlichungstag und Bekanntmachung des Hinweises auf die Patenterteilung: **02.08.2023**
(21) Anmeldenummer: 18728348.6
(22) Anmeldetag: 29.05.2018
(51) Int. Cl.: A61B 5/00

(54) **HAARBEHANDLUNGSVORRICHTUNG, HAARBEHANDLUNGSSYSTEM UND VERFAHREN ZUM KOSMETISCHEN BEHANDELN VON HAAREN**
HAIR TREATMENT APPARATUS, HAIR TREATMENT SYSTEM, AND METHOD FOR COSMETICALLY TREATING HAIR
DISPOSITIF ET SYSTÈME DE TRAITEMENT DES CHEVEUX ET PROCÉDÉ DE TRAITEMENT COSMÉTIQUE DES CHEVEUX

(30) Priorität: 01.06.2017 DE 102017209339
(43) Veröffentlichungstag der Anmeldung: 08.04.2020
(73) Patentinhaber: Henkel AG & Co. KGaA, 40589 Düsseldorf (DE)
(72) Erfinder: MATHIASZYK, Carsten, 45277 Essen (DE); LECHNER, Torsten, 40764 Langenfeld (DE)
(86) Internationale Anmeldenummer: PCT/EP2018/063993
(87) Internationale Veröffentlichungsnummer: WO 2018/219899

(56) Entgegenhaltungen:
- US-A1- 2012 227 758
- US-A1- 2015 342 515
- US-A1- 2016 286 927

## Beschreibung

Die Erfindung betrifft Haarkosmetik, insbesondere eine Haarbehandlungsvorrichtung, ein Haarbehandlungssystem und ein Verfahren zum kosmetischen Behandeln von Haaren.

Nutzer (auch als Verbraucher bezeichnet) wissen oft nicht, in welchem Maße ihre Haare geschädigt sind, und wissen auch sonst oft wenig über den Zustand ihrer Haare. Der Haarzustand, und insbesondere der Haarschädigungsgrad, kann je nach Behandlungshistorie und Veranlagung stark variieren. Diese Information kann wichtig sein, damit ein/e richtige/s, d.h. zum Haarschädigungsgrad passende/s kosmetische Haarbehandlung und/ oder kosmetisches Haarbehandlungsmittel ausgewählt werden kann/können.

Bereiche der Haarkosmetik betreffen beispielsweise Haarstyling, Haarpflege, Haarumformung (z.B. Glättung und/oder Lockung, permanent, semipermanent oder temporär, wobei die temporäre Haarumformung auch als dem Haarstylingbereich zugeordnet betrachtet werden kann) und/oder Haarcoloration (auch als Haarfärbung bezeichnet, permanent oder temporär).

Das Haarstyling und die temporäre Haarverformung können im Wesentlichen auf einer Wirkung von Haarstylingmitteln beruhen. Dabei werden die Haare in der Regel oberflächlich mit einem Stylingmittel beschichtet. Diese Beschichtung bewirkt eine Änderung von Halteeigenschaften des Haares.

Die Stylingprodukte sollten an den individuellen Zustand der Haare angepasst werden bzw. unter Berücksichtigung des individuellen Zustands ausgewählt werden.

Ebenso sollten Pflegeprodukte auf den individuellen Zustand der Haare angepasst werden. Häufig verwenden Nutzer falsche, d.h. nicht auf ihren Haarzustand abgestimmte Pflegeprodukte, die die Haare beispielsweise beschweren oder schwer handhabbar machen.

Bei der semipermanenten Haarverformung wird zur Erzielung des kosmetischen Effekts in die Haarstruktur eingegriffen. Dies geschieht beispielsweise durch ein Erhitzen des Haares mittels eines Glätteisens oder eines Lockenstabs. Dieser Vorgang kann aufgrund der einwirkenden hohen Temperaturen (120°C bis 240°C) für das Haar möglicherweise nicht unschädlich sein. Insbesondere vorgeschädigtes Haar kann bei einer falsch angewendeten semipermanenten Haarverformung, beispielsweise bei zu starkem Hitzeeintrag durch ein Glätteisen, stark (weiter) geschädigt werden.

Die genaue Abstimmung der bei der Glättung eingesetzten Temperaturen und/oder kosmetischen Pflegemittel kann daher bei der semipermanenten Haarverformung eine noch größere Bedeutung haben als bei den weiter oben beschriebenen Pflege- und temporären Haarverformungsverfahren. Hinsichtlich der Haarcoloration kann geschädigtes, bereits gefärbtes und/oder graues Haar bei Färbungen (beispielsweise bei Färbungen, die der Nutzer selbst vornimmt) oftmals zu Abweichungen von einem gewünschten Färbeergebnis führen.

Dokument US2012227758A1 beschreibt ein Haarstylinggerät mit verbesserter Steuerung der abgegebenen Temperatur. Das Dokument US 2015342515A1 beschreibt eine angeschlossene Haarbürste, die in der Lage ist, verschiedene Haareigenschaften mithilfe von Sensoren zu bestimmen.

Es besteht somit ein Bedarf daran, einem Nutzer gezielte und individuellen Pflegeanweisungen im Bereich der Haarpflege, des Haarstylings, der Haarcoloration und/oder der Haarumformung bereitzustellen.

Ferner besteht beim Nutzer ein Bedarf an einfacheren und weniger zeitintensiven kosmetischen Verfahren.

Außerdem wäre es wünschenswert, dem Nutzer während einer kosmetischen Haarbehandlung eine objektive Beurteilung eines Behandlungserfolgs und/oder eines Behandlungsverlaufs zu ermöglichen. Ebenso wäre es wünschenswert, dem Nutzer Hilfestellung bei der Durchführung einer kosmetischen Haarbehandlung zu geben.

In verschiedenen Ausführungsbeispielen wird erfindungsgemäß eine Haarbehandlungsvorrichtung bereitgestellt welche mittels mindestens eines integrierten Sensors einen Haarzustand ermittelt und, basierend auf dem ermittelten Haarzustand, einen Haar-Behandlungsparameter steuert und mindestens ein Haar-Behandlungsmittel dosiert und optional eine Haarbehandlungsempfehlung bereitstellt und welche mittels mindestens eines weiteren integrierten Sensors Bewegungen und Ortsveränderungen ermittelt und basierend auf den ermittelten Bewegungen und Ortsveränderungen eine räumliche Lage und/oder eine Geschwindigkeit ermittelt. In verschiedenen Ausführungsbeispielen kann der Haar-Behandlungsparameter ein Parameter sein, der an der Haarbehandlungsvorrichtung einstellbar ist, beispielsweise eine Temperatur der Haarbehandlungsvorrichtung. In verschiedenen Ausführungsbeispielen kann das Haar-Behandlungsmittel mittels der Haarbehandlungsvorrichtung aufbringbar sein, beispielsweise auf das Haar und/oder auf die Kopfhaut, und eine Dosierung, z.B. mittels Ventilen, kann mittels der Haarbehandlungsvorrichtung erfolgen. In verschiedenen Ausführungsbeispielen kann die Haarbehandlungsempfehlung sich auf Haar-Behandlungsparameter einer mittels der Haarbehandlungsvorrichtung vorgenommenen Haarbehandlung beziehen, welche einer direkten Steuerung mittels der Haarbehandlungsvorrichtung nicht zugänglich sind, beispielsweise eine Empfehlung, welches Haar-Behandlungsmittel in Tanks der Haarbehandlungsvorrichtung einzufüllen sind und/oder mit welcher Geschwindigkeit entlang des Haars eine Haarbehandlung zu erfolgen hat, oder ähnliches.

In verschiedenen Ausführungsbeispielen kann bei einer semipermanenten Umformung der Haare eine Abgabe von Pflege- und/oder Stylingmitteln und eine Erhitzung der Haare in einem Verfahrensschritt vereint werden.

In verschiedenen Ausführungsbeispielen werden Verfahren zur semipermanenten Haarverformung, temporären Haarverformung und Haarpflege bereitgestellt.

Im Bereich der semipermanenten Haarverformung werden in verschiedenen Ausführungsbeispielen Verfahren unter Verwendung stromführender Kleingeräte wie Glätteisen, Lockenstäben oder Trockenhauben bereitgestellt, sowie die dafür geeigneten Haarbehandlungsvorrichtungen und Haarbehandlungssysteme.

Im Bereich der temporären Haarverformung und Haarpflege werden in verschiedenen Ausführungsbeispielen Verfahren unter Verwendung stromführender Kleingeräte wie Kämmen oder Bürsten bereitgestellt, sowie die dafür geeigneten Haarbehandlungsvorrichtungen und Haarbehandlungssysteme.

In verschiedenen Ausführungsbeispielen werden Verfahren bereitgestellt, welche eine Kombination aus einem Stylinggerät (z.B. Kamm, Bürste, Lockenstab, Trockenhaube, insbesondere Glätteisen), einer Sensorik, d.h. mindestens einen Sensor, wobei der mindestens eine Sensor in das Stylinggerät integriert oder nicht in das Stylinggerät integriert sein kann, einem Prozessor und einem Aktuator nutzen.

Ferner werden Haarbehandlungsvorrichtungen und Haarbehandlungssysteme bereitgestellt, welche eine Kombination aus einem Stylinggerät (z.B. Kamm, Bürste, Lockenstab, Trockenhaube, insbesondere Glätteisen), einer Sensorik, d.h. mindestens einen Sensor, wobei der mindestens eine Sensor in das Stylinggerät integriert oder nicht in das Stylinggerät integriert sein kann, einem Prozessor und einem Aktuator aufweisen.

In verschiedenen Ausführungsbeispielen kann der Prozessor eingerichtet sein, Sensordaten vom Sensor zu empfangen, die Sensordaten auszuwerten und gegebenenfalls die ausgewerteten Sensordaten mit mindestens einer externen Datenbank (z.B. mittels einer Cloud) abzugleichen.

In verschiedenen Ausführungsbeispielen kann der Prozessor ferner eingerichtet sein, aus den ausgewerteten Sensordaten Handlungsanweisungen abzuleiten (z.B. ein Verfahrensprofil einschließlich Temperatur und/oder eingesetzten Pflege- und/oder Stylingmitteln).

In verschiedenen Ausführungsbeispielen kann der Prozessor ferner eingerichtet sein, gegebenenfalls Handlungsanweisung an den Aktuator zu übermitteln, wobei der Aktuator Teil der Haarbehandlungsvorrichtung bzw. des Haarbehandlungssystems sein kann.

In verschiedenen Ausführungsbeispielen kann der Aktuator die vom Prozessor bereitgestellte Handlungsanweisung umsetzen. Eine vom Aktuator ausgeführte Handlung kann in verschiedenen Ausführungsbeispielen eine optische Wirkung haben (z.B. als Warnlicht, Bild, Diagramm, Piktogramm, auf einem Bildschirm, z.B. dem Bildschirm eines Smartphones, dargestellter Film, o.ä.). In verschiedenen Ausführungsbeispielen kann die vom Aktuator ausgeführte Handlung eine mechanische Wirkung haben (z.B. als automatische Temperatureinstellung und/oder automatische Dosiereinstellung am Stylinggerät und/oder eine Vibration). In verschiedenen Ausführungsbeispielen kann die vom Aktuator ausgeführte Handlung eine akustische Wirkung haben (z.B. als Warnton, Sprachausgabe oder ähnliches).

In verschiedenen Ausführungsbeispielen kann der Sensor zum Erfassen eines Haarzustandsparameters einen Haarschädigungssensor aufweisen. Der Haarschädigungssensor kann in verschiedenen Ausführungsbeispielen eingerichtet sein, mittels einer Nahinfrarot-Spektroskopie und/oder einer Fluoreszenzspektroskopie einen Gehalt an oxidativen und/oder chemischen Abbauprodukten von Haarinhaltsstoffen, insbesondere einen Cysteinsäuregehalt, des Haars zu ermitteln und daraus einen Haarschädigungsgrad des Haars zu ermitteln. Der Haarschädigungssensor kann in verschiedenen Ausführungsbeispielen eingerichtet sein, während eines Kämmens des Haars erfasste akustische Emissionen aufzunehmen und anhand dessen den Haarschädigungsgrad des Haars zu ermitteln, ggf. unter Zuhilfenahme des Prozessors.

In verschiedenen Ausführungsbeispielen kann der Haarschädigungssensor einen mikroskopischen Fotosensor aufweisen. Der mikroskopische Fotosensor kann eingerichtet sein, eine Haaroberflächenrauigkeit zu ermitteln oder das Ermitteln der Haaroberflächenrauigkeit zu ermöglichen.

In verschiedenen Ausführungsbeispielen kann der Sensor zum Erfassen eines Haarzustandsparameters einen Haardickesensor aufweisen. Der Haardickesensor kann in verschiedenen Ausführungsbeispielen eingerichtet sein, mittels eines Lichtsensors eine Haardicke zu ermitteln. Beispielsweise kann bei dem Ermitteln der Haardicke berücksichtigt werden, dass dickeres Haar mehr Licht absorbiert. Der Haardickesensor kann dafür in verschiedenen Ausführungsbeispielen derart eingerichtet sein, dass in ein vorgegebenes Volumen eine vorbestimmte Menge an Haar, z.B. einlagig, einbringbar ist, und das Volumen von Licht mit einer vorbestimmten Intensität bestrahlt wird, wobei die Lichtmenge, die nach dem Durchstrahlen des Haars den Sensor zum Erfassen eines Haarzustandsparameters erreicht, mittels des Sensors gemessen werden kann. Mittels des Haardickesensors kann, ggf. in Verbindung mit dem Prozessor, anhand des erfassten Lichts die Haardicke ermittelt werden. In verschiedenen Ausführungsbeispielen kann der Haardickesensor, z.B. in einem Fall, dass der Haardickesensor eine Farbkamera aufweist, außerdem für ein Ermitteln einer Haarfarbe und/oder eines Grauanteils genutzt werden, ggf. unter Zuhilfenahme des Prozessors.

In verschiedenen Ausführungsbeispielen kann der Haardickesensor einen Ultraschallsensor aufweisen. Der Ultraschallsensor kann eingerichtet sein, Ultraschallwellen in Richtung zu den Haaren abzustrahlen, von den Haaren reflektierte Ultraschallwellen zu erfassen und daraus, ggf. in Verbindung mit dem Prozessor, die Haardicke zu ermitteln.

In verschiedenen Ausführungsbeispielen kann der mindestens eine Sensor zum Erfassen eines Haarzustandsparameters einen Haarlängesensor aufweisen. Der Haarlängesensor kann beispielsweise mindestens einen Lagesensor aufweisen, der eine Bestimmung einer Wegstrecke ermöglicht, welche im Haar zurückgelegt wurde. In verschiedenen Ausführungsbeispielen kann der Haarlängesensor kombiniert sein mit einem Sensor zum Berechnen einer Kämmbarkeit des Haars (siehe unten).

In verschiedenen Ausführungsbeispielen kann anstelle eines Ermittelns der Haarlänge mittels des Haarlängesensors die Haarlänge vom Nutzer bereitgestellt werden oder sein. Beispielsweise kann der Nutzer die Haarlänge selbst ausmessen und den gemessenen Haarlängewert der Haarbehandlungsvorrichtung bzw. dem Haarbehandlungssystem bereitstellen.

In verschiedenen Ausführungsbeispielen kann der Haardickesensor einen photooptischen Sensor aufweisen, bei welchem ein Bild von mindestens einem Haar aufgenommen wird.

In verschiedenen Ausführungsbeispielen kann der Haardickesensor einen thermischen Sensor aufweisen.

In verschiedenen Ausführungsbeispielen kann der mindestens eine Sensor zum Erfassen eines Haarzustandsparameters einen Grauanteil-Sensor aufweisen. Von dem mindestens einen Grauanteil-Sensor kann mittels eines optischen Sensors, z.B. mittels einer Kamera, ein Bild der Haare aufgenommen werden. Zum Ermitteln des Grauanteils der Haare kann das Bild in verschiedenen Ausführungsbeispielen mit mindestens einem vorhandenen Bild, z.B. einem Referenzbild, welches beispielsweise intern und/oder extern gespeichert sein kann, verglichen werden, um den Grauanteil zu ermitteln.

In verschiedenen Ausführungsbeispielen kann der Sensor zum Erfassen eines Haarzustandsparameters einen Glätte-/Lockigkeitssensor aufweisen, der eingerichtet sein kann, eine Haarstruktur im Sinne glatten Haars bis hin zu lockigem bzw. krausem Haar zu ermitteln. Der Glätte-/Lockigkeitssensor kann in verschiedenen Ausführungsbeispielen eine Kamera aufweisen. Der Glätte-/Lockigkeitssensor kann in verschiedenen Ausführungsbeispielen eingerichtet sein, ggf. in Verbindung mit dem Prozessor, z.B. mittels eines Bildverarbeitungsprogramms, eine Glätte bzw. eine Lockigkeit des Haars zu ermitteln.

In verschiedenen Ausführungsbeispielen kann der Sensor zum Erfassen eines Haarzustandsparameters einen Haarfeuchtigkeitssensor aufweisen. Der Haarfeuchtigkeitssensor kann in verschiedenen Ausführungsbeispielen eingerichtet sein, einen Wassergehalt des Haars zu ermitteln. Der Haarfeuchtigkeitssensor kann beispielsweise als Nahinfrarotspektroskop gestaltet sein, welches eingerichtet sein kann, Nahinfrarot-(NIR-)Absorptionsstrukturen von Wasser zu untersuchen und anhand dessen, ggf. unter Zuhilfenahme des Prozessors, die Haarfeuchtigkeit zu ermitteln.

In verschiedenen Ausführungsbeispielen kann der mindestens eine Sensor zum Erfassen eines Haarzustandsparameters einen Kämmarbeit-Sensor aufweisen. Der Kämmarbeit-Sensor kann eingerichtet sein, einen Kraftaufwand zu erfassen (beispielsweise mittels Dehnmessstreifen), der bei einem Kämmen der Haare aufgewendet wird.

In verschiedenen Ausführungsbeispielen kann der mindestens eine Sensor zum Erfassen eines Haarzustandsparameters einen Haardichtesensor aufweisen. Der Haardichtesensor kann beispielsweise eine Kamera oder einen Kameraaufsatz aufweisen, welche/r eingerichtet sein kann, direkt an eine Haarwurzelgegend gehalten zu werden, z.B. direkt auf eine Kopfhaut aufgesetzt zu werden. Eine Haardichte kann anhand des Bildes beispielsweise anhand einer Zahl von Haaren und/oder eines Abstands zwischen den Haaren, die Haardichte zu ermitteln.

In verschiedenen Ausführungsbeispielen kann der Sensor zum Erfassen eines Haarzustandsparameters eingerichtet sein, weitere Haarzustandsparameter zu ermitteln.

In verschiedenen Ausführungsbeispielen kann der Sensor zum Erfassen eines Haarzustandsparameters, z.B. im Fall eines Spektrometers oder einer Kamera, eingerichtet sein, mehrere Haarzustandsparameter zu ermitteln, z.B. sowohl den Haarschädigungsgrad anhand der Cysteinsäure-Absorptionsstrukturen im NIR-Spektrum als auch die Haarfeuchtigkeit anhand der Wasser-Absorptionsstrukturen im NIR-Spektrum.

In verschiedenen Ausführungsbeispielen kann die Haarbehandlungsvorrichtung oder das Haarbehandlungssystem ein heizbares Gerät aufweisen oder sein. Das heißt, dass die Haarbehandlungsvorrichtung oder das Haarbehandlungssystem in verschiedenen Ausführungsbeispielen eine Heizvorrichtung aufweisen kann. Die Heizvorrichtung kann in verschiedenen Ausführungsbeispielen je nach Ergebnis der Haaranalyse (Schädigung, Haardicke, Lockigkeit, Wassergehalt) gesteuert oder geregelt werden. Beispielsweise kann die Haarbehandlungsvorrichtung oder das Haarbehandlungssystem bei intakterem, dickerem, lockigerem und/oder feuchterem Haar so gesteuert oder geregelt werden, dass das Haar mit einer höheren Temperatur behandelt wird, als wenn das Haar geschädigter, dünner, glatter und/oder trockener ist.

In verschiedenen Ausführungsbeispielen kann die Haarbehandlungsvorrichtung und/oder das Haarbehandlungssystem ein Glätteisen, einen Lockenstab und/oder eine Trockenhaube mit einer Temperatursteuerung und/oder einer Temperaturregelung aufweisen.

In verschiedenen Ausführungsbeispielen kann die Haarbehandlungsvorrichtung und/oder das Haarbehandlungssystem ein Glätteisen, einen Lockenstab und/oder eine Trockenhaube mit einem (z.B. nachfüllbaren) Tank zur Aufnahme und dosierten Abgabe eines Haarbehandlungsmittels (auch als Aktivstofftank bezeichnet) und eine Dosiervorrichtung aufweisen.

In verschiedenen Ausführungsbeispielen kann die Haarbehandlungsvorrichtung und/oder das Haarbehandlungssystem einen Kamm und/oder eine Bürste mit einem (z.B. nachfüllbaren) Tank zur Aufnahme und dosierten Abgabe eines Haarbehandlungsmittels und eine Dosiervorrichtung aufweisen.

In verschiedenen Ausführungsbeispielen kann die Haarbehandlungsvorrichtung und/oder das Haarbehandlungssystem, z.B. das Stylinggerät, die Sensorik in einer doppelten Ausführung aufweisen. Dabei kann eine Mehrzahl von (z.B. gleichartigen) Sensoren derart angeordnet sein, dass der Nutzer sofort eine Rückmeldung darüber erhalten kann, ob eine weitere Behandlung (auch als "Post-Treatment" bezeichnet) notwendig ist. In verschiedenen Ausführungsbeispielen kann die Haarbehandlungsvorrichtung und/oder das Haarbehandlungssystem, z.B. das Stylinggerät, auf beiden Seiten der Heizvorrichtung Sensoren aufweisen, wobei ein Sensor zum Erfassen eines Haarzustandsparameters einen Haarzustandsparameter vor und ein Sensor zum Erfassen eines Haarzustandsparameters einen (z.B. denselben) Haarzustandsparameter nach der Temperaturbehandlung der Haare misst.

In verschiedenen Ausführungsbeispielen kann die Haarbehandlungsvorrichtung und/oder das Haarbehandlungssystem, z.B. das Stylinggerät, alternativ oder zusätzlich eine Abgabevorrichtung aufweisen. In der Abgabevorrichtung kann sich ein Haar-Behandlungsmittel, z.B. ein Pflege- oder Stylingmittel, befinden. Je nach Ergebnis der Haaranalyse (Schädigung, Haardicke, Lockigkeit, Wassergehalt) kann das Haarbehandlungsmittel, das z.B. eine (z.B. chemische) Zusammensetzung aufweisen kann, gegebenenfalls in unterschiedlichen Volumina/Mengen je nach Position [Ansatz, Mitte, Spitzen] auf das Haar aufgebracht werden.

In verschiedenen Ausführungsbeispielen können mittels der Haarbehandlungsvorrichtung und/oder des Haarbehandlungssystems zwei oder mehr Mittel in unterschiedlichen Mischungsverhältnissen je nach Position [Ansatz, Mitte, Spitzen] oder an unterschiedlichen Positionen auf das Haar aufgebracht werden oder auf das Haar aufbringbar sein.

In verschiedenen Ausführungsbeispielen kann die Haarbehandlungsvorrichtung und/oder das Haarbehandlungssystem eine Abgabevorrichtung aufweisen, welche für eine Abgabe der Mittel Pumpen aufweisen kann, welche beispielsweise mittels drahtloser Anweisungen (z.B. mittels WLAN, Bluetooth, o.ä. übertragenen Anweisungen), die vom Prozessor gesendet werden können, gesteuert werden oder sein. Beispielsweise kann eine Flussrate des Mittels bzw. der Mittel eingestellt werden oder sein.

In verschiedenen Ausführungsbeispielen kann ein Nutzer Informationen über einen Zustand seiner Haare erhalten (auch als Haarstatus bezeichnet).

In verschiedenen Ausführungsbeispielen kann ein Nutzer eine auf seinen Haarstatus abgestimmte personalisierte kosmetische Behandlung und/oder Behandlungsempfehlung erhalten.

In verschiedenen Ausführungsbeispielen kann ein Nutzer bereits während einer Durchführung einer Haarbehandlung Informationen über einen Verlauf der Anwendung erhalten. Ein Haarbehandlungsergebnis kann noch während der Durchführung der Haarbehandlung optimiert werden, so dass Frustrationen vermieden werden können.

In verschiedenen Ausführungsbeispielen kann ein zeitsparendes Haarbehandlungsverfahren bereitgestellt werden, beispielsweise indem eine (temporäre, semipermanente oder permanente) Haarumformungsbehandlung zumindest teilweise zusammen mit einer Haarpflegebehandlung erfolgt.

In verschiedenen Ausführungsbeispielen kann eine Haarbehandlungsvorrichtung in Form eines Glätteisens bereitgestellt werden, bei welchem der mindestens eine Sensor zum Erfassen eines Haarzustandsparameters in das Glätteisen integriert ist, eine elektronische Schaltkreisvorrichtung, die einen Prozessor aufweisen kann, in das Glätteisen integriert ist, und ein erster Aktuator, der beispielsweise eine Temperatursteuerung oder eine Temperaturregelung aufweisen kann, welche eine Temperatur beheizbarer Flächen des Glätteisens steuert oder regelt, in das Glätteisen integriert ist. Darüber hinaus kann in verschiedenen Ausführungsbeispielen das Glätteisen einen zweiten Aktuator aufweisen, der beispielsweise einen Dosierer für ein Haarpflege- und/oder Haarstylingmittel aufweisen kann.

In verschiedenen Ausführungsbeispielen kann ein Haarbehandlungssystem in Form eines Glätteisens bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung oder ein so genannter "akustischer Kamm", welcher eingerichtet sein kann, beim Kämmen entstehende Geräusche zu erfassen), eine elektronische Schaltkreisvorrichtung, die einen Prozessor aufweisen kann, in das Glätteisen integriert ist, und ein erster Aktuator, der beispielsweise eine Temperatursteuerung oder eine Temperaturregelung aufweisen kann, welche eine Temperatur beheizbarer Flächen des Glätteisens steuert oder regelt, in das Glätteisen integriert ist. Darüber hinaus kann in verschiedenen Ausführungsbeispielen das Glätteisen einen zweiten Aktuator aufweisen, der beispielsweise einen Dosierer für ein Haarpflege- und/oder Haarstylingmittel aufweisen kann. In verschiedenen Ausführungsbeispielen kann das Glätteisen eine Datenaustauschvorrichtung aufweisen, beispielsweise zum Empfangen von mittels des mindestens einen Sensors erfassten Messwerten.

In verschiedenen Ausführungsbeispielen kann ein Haarbehandlungssystem in Form eines Glätteisens bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung oder ein so genannter "akustischer Kamm", welcher eingerichtet sein kann, beim Kämmen entstehende Geräusche zu erfassen), eine elektronische Schaltkreisvorrichtung, die einen Prozessor aufweisen kann, in die erste separate kommunikationsfähige Vorrichtung integriert ist, und ein erster Aktuator, der beispielsweise eine Temperatursteuerung oder eine Temperaturregelung aufweisen kann, welche eine Temperatur beheizbarer Flächen des Glätteisens steuert oder regelt, in das Glätteisen integriert ist. Darüber hinaus kann in verschiedenen Ausführungsbeispielen das Glätteisen einen zweiten Aktuator aufweisen, der beispielsweise einen Dosierer für ein Haarpflege- und/oder Haarstylingmittel aufweisen kann. In verschiedenen Ausführungsbeispielen kann das Glätteisen eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem in Form eines Glätteisens bereitgestellt werden, bei welchem der mindestens eine Sensor zum Erfassen eines Haarzustandsparameters bei welchem der mindestens eine Sensor zum Erfassen eines Haarzustandsparameters in das Glätteisen integriert ist, eine elektronische Schaltkreisvorrichtung, die einen Prozessor aufweisen kann, Teil einer zweiten separaten kommunikationsfähigen (d.h. eine Datenaustauschvorrichtung aufweisenden) Vorrichtung ist (beispielsweise Teil eines Smartphones, auf dem beispielsweise eine App installiert sein kann, oder beispielsweise eine Cloud oder ähnliches), und ein erster Aktuator, der beispielsweise eine Temperatursteuerung oder eine Temperaturregelung aufweisen kann, welche eine Temperatur beheizbarer Flächen des Glätteisens steuert oder regelt, in das Glätteisen integriert ist. Darüber hinaus kann in verschiedenen Ausführungsbeispielen das Glätteisen einen zweiten Aktuator aufweisen, der beispielsweise einen Dosierer für ein Haarpflege- und/oder Haarstylingmittel aufweisen kann. In verschiedenen Ausführungsbeispielen kann das Glätteisen eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem in Form eines Glätteisens bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung oder ein so genannter "akustischer Kamm", welcher eingerichtet sein kann, beim Kämmen entstehende Geräusche zu ermitteln), eine elektronische Schaltkreisvorrichtung, die einen Prozessor aufweisen kann, Teil einer zweiten separaten kommunikationsfähigen (d.h. eine Datenaustauschvorrichtung aufweisenden) Vorrichtung ist (beispielsweise Teil eines Smartphones, auf dem beispielsweise eine App installiert sein kann, oder beispielsweise eine Cloud oder ähnliches), und ein erster Aktuator, der beispielsweise eine Temperatursteuerung oder eine Temperaturregelung aufweisen kann, welche eine Temperatur beheizbarer Flächen des Glätteisens steuert oder regelt, in das Glätteisen integriert ist. Darüber hinaus kann in verschiedenen Ausführungsbeispielen das Glätteisen einen zweiten Aktuator aufweisen, der beispielsweise einen Dosierer für ein Haarpflege- und/oder Haarstylingmittel aufweisen kann. In verschiedenen Ausführungsbeispielen kann das Glätteisen eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann eine Haarbehandlungsvorrichtung in Form eines Kamms bereitgestellt werden, bei welchem der mindestens eine Sensor zum Erfassen eines Haarzustandsparameters (beispielsweise in Form eines Mikrofons oder einer Kamera zum Erfassen von Haarzustandsparametern) in den Kamm integriert ist, eine elektronische Schaltkreisvorrichtung, die einen Prozessor aufweisen kann, in den Kamm integriert ist, wobei der Prozessor eingerichtet sein kann, mittels der erfassten Haarzustandsparameter beispielsweise einen Haarschädigungsgrad und basierend darauf mindestens einen Steuerungsparameter und ggf. mindestens eine Empfehlung zu ermitteln, und ein erster Aktuator, der beispielsweise einen Dosierer für ein Haarpflege- und/oder Haarstylingmittel aufweisen kann, in den Kamm integriert ist oder mit dem Kamm zu einem integrierten System verbindbar ist, beispielsweise in Form eines Aufsatzes für den Kamm.

In verschiedenen Ausführungsbeispielen kann ein Haarbehandlungssystem in Form eines Kamms bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung oder ein so genannter "akustischer Kamm"), eine elektronische Schaltkreisvorrichtung, die einen Prozessor aufweisen kann, in den Kamm integriert ist, wobei der Prozessor eingerichtet sein kann, mittels der erfassten Haarzustandsparameter beispielsweise einen Haarschädigungsgrad und basierend darauf mindestens einen Steuerungsparameter und ggf. mindestens eine Empfehlung zu ermitteln, und ein erster Aktuator, der beispielsweise einen Dosierer für ein Haarpflege- und/oder Haarstylingmittel aufweisen kann, in den Kamm integriert ist oder mit dem Kamm zu einem integrierten System verbindbar ist, beispielsweise in Form eines Aufsatzes für den Kamm. In verschiedenen Ausführungsbeispielen kann der Kamm eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem in Form eines Kamms bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung oder ein so genannter "akustischer Kamm"), eine elektronische Schaltkreisvorrichtung, die einen Prozessor aufweisen kann, in die erste separate kommunikationsfähige Vorrichtung integriert ist, und ein erster Aktuator, der beispielsweise einen Dosierer für ein Haarpflege- und/oder Haarstylingmittel aufweisen kann, in den Kamm integriert ist oder mit dem Kamm zu einem integrierten System verbindbar ist, beispielsweise in Form eines Aufsatzes für den Kamm. In verschiedenen Ausführungsbeispielen kann der Kamm eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem in Form eines Kamms bereitgestellt werden, bei welchem der mindestens eine Sensor zum Erfassen eines Haarzustandsparameters in den Kamm integriert ist, eine elektronische Schaltkreisvorrichtung, die einen Prozessor aufweisen kann, Teil einer zweiten separaten kommunikationsfähigen (d.h. eine Datenaustauschvorrichtung aufweisenden) Vorrichtung ist (beispielsweise Teil eines Smartphones, auf dem beispielsweise eine App installiert sein kann, oder beispielsweise eine Cloud oder ähnliches), und ein erster Aktuator, der beispielsweise einen Dosierer für ein Haarpflege- und/oder Haarstylingmittel aufweisen kann, in den Kamm integriert ist oder mit dem Kamm zu einem integrierten System verbindbar ist, beispielsweise in Form eines Aufsatzes für den Kamm. In verschiedenen Ausführungsbeispielen kann der Kamm eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem in Form eines Kamms bereitgestellt werden, bei welchem der mindestens eine Sensor zum Erfassen eines Haarzustandsparameters Teil einer ersten separaten kommunikationsfähigen (d.h. eine Datenaustauschvorrichtung aufweisenden) Vorrichtung ist (beispielsweise ein Smartphone, das eine Kamera aufweist, ein Spektrometer mit der Datenaustauschvorrichtung, ein so genannter "akustischer Kamm"), eine elektronische Schaltkreisvorrichtung, die einen Prozessor aufweisen kann, Teil einer zweiten separaten kommunikationsfähigen (d.h. eine Datenaustauschvorrichtung aufweisenden) Vorrichtung ist (beispielsweise Teil eines Smartphones, auf dem beispielsweise eine App installiert sein kann, oder beispielsweise eine Cloud oder ähnliches), und ein erster Aktuator, der beispielsweise einen Dosierer für ein Haarpflege- und/oder Haarstylingmittel aufweisen kann, in den Kamm integriert ist oder mit dem Kamm zu einem integrierten System verbindbar ist, beispielsweise in Form eines Aufsatzes für den Kamm. In verschiedenen Ausführungsbeispielen kann der Kamm eine Datenaustauschvorrichtung aufweisen, beispielsweise zum Empfangen von mittels der elektronischen Schaltkreisvorrichtung ermittelten Empfehlungen und/oder Steuerungsanweisungen.

In verschiedenen Ausführungsbeispielen kann ein Haarbehandlungssystem bereitgestellt werden, welches eine Haarbehandlungsvorrichtung (z.B. ein Glätteisen, einen Lockenstab, einen Kamm, eine Bürste o.ä.) aufweisen kann, und ferner eine Haarzustands-Ermittlungsvorrichtung, welche eingerichtet sein kann, einen Haarschädigungsgrad zu erfassen, beispielsweise mittels mindestens eines in die Haarzustands-Ermittlungsvorrichtung integrierten Sensors (die Haarzustands-Ermittlungsvorrichtung kann beispielsweise einen mit Sensoren ausgestatteten Kamm oder eine NIR-Kamera oder ein NIR-Spektrometer oder ein UV-Spektrometer oder ein UV/VIS-Spektrometer oder ein VIS/NIR-Spektrometer aufweisen, welche/r/s geeignet sein kann, einen Haarzustandsparameter, z.B. eine Haarstruktur, zu ermitteln). Über eine Smartphone-App, die als Herzstück des Systems fungieren kann, können dann gezielt Informationen, z.B. Steuerungs- bzw. Regelungsanweisungen, an die Haarbehandlungsvorrichtung (z.B. ein "smartes" Glätteisen) übertragen werden, welche eingerichtet sein kann, anhand der Steuerungs- bzw. Regelungsanweisungen einen Haarbehandlungsparameter zu steuern bzw. regeln und/oder ein Haarbehandlungsmittel zu dosieren (im Fall des Glätteisens kann z.B. eine Temperatur eingestellt werden, mit der die Haare ohne weitere Schädigung behandelt werden können). Ein Datenaustausch kann hierbei kabellos erfolgen, z.B. über Bluetooth, WLAN oder eine Near Field Communication Technologie (NFC-Technologie).

In verschiedenen Ausführungsbeispielen kann ein Haarbehandlungssystem bereitgestellt werden, welches eine Haarbehandlungsvorrichtung (z.B. ein Glätteisen, einen Lockenstab, einen Kamm, eine Bürste o.ä.) aufweisen kann, und ferner eine Datenverbindung (für die Möglichkeit des Datenaustauschs ist auch der Begriff Konnektivität bzw. Connectivity gebräuchlich) aufweisen kann zwischen einer externen App und der Haarbehandlungsvorrichtung (z.B. einem Haarpflege/stylingGerät). In verschiedenen Ausführungsbeispielen können Pflege/Styling-Parametern bereitgestellt, z.B. vorgegeben, werden (in einem Fall, dass die Haarbehandlungsvorrichtung das Glätteisen oder den Lockenstab aufweist, kann der Parameter z.B. eine maximale Temperatur aufweisen), wobei der bereitgestellte Parameter bezogen sein kann auf einen Schädigungsgrad des Haares, d.h. je nach Schädigungsgrad des Haares kann der Parameter einen anderen Wert aufweisen.

In verschiedenen Ausführungsbeispielen kann eine Temperatur eines Glätteisens gesteuert oder geregelt sein bzw. werden unter Berücksichtigung eines Schädigungsgrads des Haars.

In verschiedenen Ausführungsbeispielen kann ein "smartes" Stylinggerät (z.B. ein Kamm, eine Bürste, ein Lockenstab, ein Glätteisen oder ähnliches) und/oder ein "smartes" Endgerät (z.B. ein Smartphone, ein Tablet, ein Smart Mirror oder ähnliches) einen Sensor zum Erfassen eines Haarzustandsparameters aufweisen, welcher in das Stylinggerät und/oder das Endgerät integriert und/oder als entfernbarer Aufsatz und/oder als eine separate tragbare Vorrichtung gestaltet sein kann. Das Stylinggerät und/oder das Endgerät kann eingerichtet sein, einen oder mehrere Haarzustandsparameter, z.B. Haareigenschaften, zu erfassen. Die erfassten Daten können mittels des Stylinggeräts und/oder des Endgeräts, beispielsweise mittels einer App und/oder eines Programms, welches auf dem Stylinggerät und/oder dem Endgerät installiert sein kann, ausgewertet werden, und dem Nutzer kann eine (personalisierte) Empfehlung bereitgestellt werden, beispielsweise in Form so genannter DOs (Empfehlungen) und DON'Ts (Warnungen). Ein Beispiel dafür wäre "Nimm (k)ein wachshaltiges Produkt". In verschiedenen Ausführungsbeispielen kann das Bereitstellen der Empfehlung optisch erfolgen, z.B. mittels einer Anzeigevorrichtung, z.B. mittels eines Displays. In verschiedenen Ausführungsbeispielen kann das Bereitstellen der Empfehlung alternativ oder zusätzlich akustisch erfolgen, beispielsweise als Sprachausgabe. In verschiedenen Ausführungsbeispielen kann das Bereitstellen der Empfehlung mittels des Stylinggeräts und/oder mittels des Endgeräts erfolgen.

In verschiedenen Ausführungsbeispielen kann die Haarbehandlungsvorrichtung bzw. das Haarbehandlungssystem eine Eingabevorrichtung aufweisen. Die Eingabevorrichtung kann eingerichtet sein, mindestens eine Eingabe durch den Nutzer aufzunehmen und der elektronischen Schaltkreisvorrichtung und/oder einer externen Datenverarbeitungsvorrichtung bereitzustellen.

Vom Nutzer einzugebende Parameter können beispielsweise eine Wunschhaarfarbe, eine Wunschbehandlung (z.B. Haarumformung, Styling, Pflege), eine Haarlänge, eine Haarlockigkeit (für eine Eingabe können beispielsweise Vergleichsbilder bereitgestellt werden) oder ähnliches aufweisen.

Die Haarbehandlungsvorrichtung weist ferner mindestens einen im oder am Vorrichtungskörper angeordneten Sensor zum Erfassen von Bewegungen und Ortsveränderungen des Vorrichtungskörpers auf.

Mit Hilfe der im oder am Vorrichtungskörper angeordneten elektronischen Schaltkreisvorrichtung, welche mit dem mindestens einen Sensor zum Erfassen von Bewegungen und Ortsveränderungen des Vorrichtungskörpers gekoppelt ist und die erfassten Sensordaten empfängt, wird basierend auf den empfangenen erfassten Sensordaten eine räumliche Lage des Vorrichtungskörpers und/oder die Geschwindigkeit des Vorrichtungskörpers ermittelt.

In verschiedenen Ausführungsbeispielen ermittelt die elektronische Schaltkreisvorrichtung ferner, ob die ermittelte Geschwindigkeit des Vorrichtungskörpers ein vorgegebenes Kriterium gemäß einer vorgesehenen Geschwindigkeit zum Anwenden der Haarbehandlungsvorrichtung erfüllt.

In Abhängigkeit davon, ob die ermittelte Geschwindigkeit das vorgegebene Kriterium erfüllt ist die elektronische Schaltkreisvorrichtung eingerichtet, ein Signal abzugeben. Das Signal kann ein akustisches Signal, ein optisches Signal und/oder haptisches Signal umfassen. Beispielsweise kann bei einer zu langsamen Bewegung der Haarbehandlungsvorrichtung durch das Haar der Anwender durch ein Tonsignal, ein Lichtsignal oder ein Vibrieren eines Teils der Haarbehandlungsvorrichtung, beispielsweise eines Griffs, darauf hingewiesen werden.

In verschiedenen Ausführungsbeispielen erfolgt die Abgabe des Signals während der Anwendung der Haarbehandlungsvorrichtung, vorzugsweise mit einer Reaktionszeit von kleiner 1 Sekunde zur Erfassung der Sensordaten bezüglich der Bewegungen und Ortsveränderung des Vorrichtungskörpers.

In verschiedenen Ausführungsbeispielen ermittelt die elektronische Schaltkreisvorrichtung basierend auf der ermittelten räumlichen Lage des Vorrichtungskörpers, welche Haarbereiche bereits mit der Haarbehandlungsvorrichtung behandelt wurden, und übermittelt die ermittelten Haarbereiche an eine Anzeigevorrichtung, welche vorzugsweise Bestandteil eines Computers, eines Smartphones, eines Tablets, eines Smart Mirrors, einer Smart Watch oder eines Laptop ist. Die Übermittlung der Daten kann hierbei kabellos erfolgen, z.B. über Bluetooth, WLAN, ZigBee, Thread oder eine Near Field Communication Technologie (NFC-Technologie).

Auf der Anzeigevorrichtung können die ermittelten, bereits behandelten Haarbereiche in einer Darstellung des Nutzers (z.B. einer schematischen Darstellung oder auf einem Foto des Nutzers) angezeigt werden. Beispielsweise können in einer schematischen Darstellung der Frisur des Nutzers bereits mit der Haarbehandlungsvorrichtung behandelte und nicht behandelte Bereiche mit unterschiedlichen Farben und/oder Mustern dargestellt werden, z.B. bereits behandelte Bereiche grün und nicht behandelte Bereiche rot oder ähnliches. In einem anderen Beispiel können einem Foto des Nutzers, z.B. einem digitalen Foto, welches die Haare oder Frisur des Nutzers zeigt, unterschiedliche Muster überlagert sein, z.B. ein Punktmuster für bereits behandelte Bereiche und ein Linienmuster für nicht behandelte Bereiche oder ähnliches. Alternativ kann die Echtzeit-Darstellung eines Nutzers auf/in einem Smart Mirror zur Anzeige der bereits behandelten und/oder der nicht-behandelten Haarbereiche verwendet werden.

In verschiedenen Ausführungsbeispielen ist der Sensor zum Erfassen von Bewegungen und Ortsveränderungen des Vorrichtungskörpers ausgewählt aus der Gruppe bestehend aus Magnetfeldsensoren, Gyroskopen, Beschleunigungssensoren und mechanisch arbeitenden Wegmesssensoren. In einer besonders bevorzugten Ausführungsform ist der Sensor zum Erfassen von Bewegungen und Ortsveränderungen des Vorrichtungskörpers ein Gyroskop.

Alternativ oder zusätzlich kann die Ermittlung der räumlichen Lage der Haarbehandlungsvorrichtung und die Ermittlung der bereits behandelten Haarbereiche mit Hilfe einer App oder einem Programm, welches auf einer separaten Datenverarbeitungsvorrichtung installiert ist, erfolgen. In diesem Fall übermittelt die elektronische Schaltkreisvorrichtung die erfassten Sensordaten zur Bewegung und Ortsveränderung des Vorrichtungskörpers, vorzugsweise kabellos, an die separate Datenverarbeitungsvorrichtung. Im Fall, dass die separate Datenverarbeitungsvorrichtung eine Anzeigevorrichtung umfasst, kann die Darstellung der bereits mit der Haarbehandlungsvorrichtung behandelten und/oder nicht behandelten Haarbereiche mit Hilfe der Anzeigevorrichtung der separaten Datenverarbeitungsvorrichtung erfolgen.

Der Sensor zum Erfassen von Bewegungen und Ortsveränderungen kann auch Bestandteil eines Haarbehandlungssystems ein. In dieser Ausführungsform kann der Sensor zum Erfassen von Bewegungen und Ortsveränderungen beispielsweise Teil einer ersten separaten kommunikationsfähigen Vorrichtung sein. Es ist besonders bevorzugt, dass bei einem Haarbehandlungssystem In Form eines Glätteisens oder eines Kamms, der Sensor zum Erfassen von Bewegungen und Ortsveränderungen in das Glätteisen oder den Kamm integriert ist.

In verschiedenen Ausführungsbeispielen kann die Haarbehandlungsvorrichtung, z.B. das smarte Stylinggerät, ferner eine Abgabevorrichtung aufweisen. In der Abgabevorrichtung, beispielsweise in mindestens einem Tank, kann in verschiedenen Ausführungsbeispielen ein Haarbehandlungsmittel angeordnet sein, z.B. ein Haarpflegemittel, ein Haarstylingmittel oder ein Haarfärbemittel. In verschiedenen Ausführungsbeispielen kann in Abhängigkeit von dem ermittelten mindestens einen Haarzustandsparameter (der ein Ergebnis einer basierend auf den Sensordaten ausgeführten Haaranalyse sein kann) das Haarbehandlungsmittel in unterschiedlichen Volumina bzw. Mengen je nach Position (z.B. Ansatz/Mitte/Spitzen) aufgebracht werden, beispielsweise ein anderes Volumen/eine andere Menge auf die Haarspitzen als auf den Haaransatz. Alternativ oder zusätzlich können zwei oder mehr Haarbehandlungsmittel in unterschiedlichen Mischungsverhältnissen je nach Position (z.B. Ansatz/Mitte/Spitzen) oder an unterschiedlichen Haarstellen (z.B. als Ombré-Färbung) auf das Haar aufgebracht werden. Die Abgabevorrichtung kann für eine Abgabe des Haarbehandlungsmittels mindestens eine Pumpe aufweisen. Die mindestens eine Pumpe kann beispielweise mittels eines Smartphones o.ä., z.B. mittels einer App, gesteuert oder geregelt werden. Beispielsweise kann mittels des Smartphones eine Flussrate des Haarbehandlungsmittels eingestellt werden.

In verschiedenen Ausführungsbeispielen kann die Abgabevorrichtung getrennt sein von der Haarbehandlungsvorrichtung.

Die Abgabevorrichtung kann in verschiedenen Ausführungsbeispielen mittels einer drahtlosen Übertragungsvorrichtung gesteuert werden, beispielsweise indem der Abgabevorrichtung Steuerbefehle mittels der drahtlosen Übertragungsvorrichtung empfängt. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN (WiFi), ZigBee, NFC, Wibree, Thread, WiMAX oder ähnlichem ermöglicht.

In verschiedenen Ausführungsbeispielen kann das Bereitstellen der Empfehlung an den Nutzer ein Bereitstellen mittels eines Übertragens der Empfehlung an eine Anzeigevorrichtung und Anzeigen der Empfehlung aufweisen.

Das Übertragen kann in verschiedenen Ausführungsbeispielen mittels einer drahtlosen Übertragungsvorrichtung erfolgen. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN (WiFi), ZigBee, NFC, Wibree, Thread, WiMAX oder ähnlichem ermöglicht.

In verschiedenen Ausführungsbeispielen kann vor einem Bereitstellen einer Empfehlung an den Nutzer ein Datenabgleich vorgenommen werden zwischen dem smarten Endgerät und/oder dem smarten Stylinggerät und Daten, z.B. Referenzdaten, welche beispielsweise in einer Cloud hinterlegt sein können. Die Daten können in verschiedenen Ausführungsbeispielen Daten von anderen Nutzern aufweisen, welche beispielsweise denselben mindestens einen Haarzustandsparameter aufweisen, und beispielsweise entsprechende abgeleitete Empfehlungen/Maßnahmen.

In verschiedenen Ausführungsbeispielen kann ein Nutzer bereits während einer Durchführung einer Haarbehandlung Informationen über einen Verlauf der Anwendung erhalten. Dadurch kann ermöglicht sein, bereits während der Durchführung ein Haarbehandlungsergebnis zu optimieren. Dadurch können Frustrationen beim Nutzer vermieden werden.

In verschiedenen Ausführungsbeispielen kann ein Nutzer Informationen über einen Haarzustand, d.h. einen Status des Haars, erhalten, was ein Erzeugen von (z.B. direkten) Anweisungen ermöglicht, beispielsweise einer Anweisung, wann ein Färbevorgang beendet werden muss (z.B. mittels rechtzeitigen Ausspülens der Haare, um ein zu dunkles Färbeergebnis zu vermeiden).

In verschiedenen Ausführungsbeispielen können die Empfehlung für Pflege- und Stylingprodukte und/oder die Anwendungshinweise sich auf eine chemische Zusammensetzung der empfohlenen und nicht zu empfehlenden Pflege- und Stylingprodukte beziehen, beispielsweise in Form so genannter DOs (Empfehlungen) und DON'Ts (Warnungen). Ein Beispiel dafür wäre "Nimm (k)ein wachshaltiges Produkt".

Je nach Haardicke und Haardichte kann dem Nutzer in verschiedenen Ausführungsbeispielen ein geeignetes Stylingprodukt empfohlen werden. Beispielsweise kann es unvorteilhaft sein, bei dünnem, lichtem Haar ein Styling-Wachs anzuwenden, da dieses bei einem solchen Haartyp schnell fettig und ungepflegt wirkt. Unter die Kategorie "Wachs" fallen Produkte, die sich durch einen hohen Anteil an Wachskomponenten, Vaselinen, Emulgatoren und Ölen bzw. öligen oder ölhaltigen Komponenten auszeichnen. Ebenfalls nicht zu empfehlen bei dünnem lichtem Haar können Emulsionen sein, die sich ebenfalls durch einen hohen Anteil eben beschriebener Rohstoffe auszeichnen können.

Je nach Eigenschaft der in den Haarpflege- und/oder Haarstylingprodukten eingesetzten Inhaltsstoffen kann die Verwendung dieser Inhaltsstoffe bei einem ermittelten Haartyp dem Nutzer empfohlen werden oder kann von einer Nutzung abgeraten werden.

Je nach Lockenzustand (eher Locken oder eher Wellen) kann dem Nutzer in verschiedenen Ausführungsbeispielen ein Produkt empfohlen werden, welches sein natürliches Lockenbild entweder unterstützt, oder im Falle von Wellen auch verstärken (auch als pushen bezeichnet) und stabilisieren kann. Bestimmte Polymere, wie bspw. Styleze CC10 (VP/DMAPA Acrylates Copolymer), Mirustyle CP (Polyquaternium-72) können hier besonders hilfreich sein. Allerdings können auch Standardpolymere, wie PVP, PVP/VA, etc. durchaus geeignet sein, den Halt der Locken zu unterstützen, und/oder weitere Polymere, deren Lockenhalte- bzw. - erzeugungsvermögen zum Zeitpunkt der Veröffentlichung dem Fachmann bekannt sind.

Locken benötigen meist viel Pflege, so dass auch bei lockigem Haar eine pflegende Emulsion/Lotion vorteilhaft sein kann. Weiterhin können Mousses angewandt werden, um Bounce und Sprungkraft zu unterstützen und die Locken zu definieren. In verschiedenen Ausführungsbeispielen können die oben für die Lockendefinition genannten Polymere genutzt werden. Die Lockendefinition kann ebenfalls mit einem leichten Wachs gelingen, wohingegen ein (schweres) Wachs bei Wellen eher beschwerend wirkt und dem Konsumenten vermutlich nicht helfen kann sein Stylingergebnis über einen längeren Zeitraum aufrechtzuerhalten. Zusätzliche Unterstützung können in verschiedenen Ausführungsbeispielen allerdings auch hier Polymere (beispielsweise wie oben genannt) liefern, um das Ergebnis und die Halteleistung zu verlängern.

Auch bei langen Haaren kann in verschiedenen Ausführungsbeispielen eine Anwendung eines Wachses oder Geles eher unüblich sein. In diesem Fall kann dem Nutzer empfohlen werden, auf leichtere Konsistenzen, wie z.B. Blow-Dry Sprays, Mousses, Lotionen o.ä. zurückgreifen, um ihren Look zu unterstützen. Besonders sprühbare oder auch niedrigviskose pumpbare Produkte können hier gut geeignet sein, da sich diese besonders leicht in der gesamten Haarlänge verteilen lassen. Je nach gewünschtem Styling Effekt kann es auch hier unterschiedliche Polymertypen geben, die zum Einsatz kommen können (z.B. PVP, PVP/VA, Chitosan, Polyquaternium-Typen, usw.).

In verschiedenen Ausführungsbeispielen kann eine Wahl des richtigen Produktes meist mit dem gewünschten Stylingergebnis zusammenhängen. So kann beispielsweise ein Blow-Dry Spray oder ein Mousse dafür vorgesehen sein, unterstützend beim Föhnen zu wirken, und kann zugleich pflegende Styligkomponenten enthalten, die ein Arbeiten mit Kamm und Bürste im feuchten Haar zulassen (bspw. Polyquaternium-4, Polyquaternium-11, Polyquaternium-46, usw., Chitosan, Polyacrylamidopropyltrimonium Chloride, usw.).

Ein Haarspray kann dafür vorgesehen sein, die finale Frisur dann noch zu fixieren und zudem möglichst davor schützen, dass äußere Einflüsse die Frisur gefährden. Ein Beispiel für einen solchen äußeren Einfluss wäre der Einfluss hoher Luftfeuchtigkeit. Bestimmte Polymer können diesem entgegenwirken, z.B. Amphomer (siehe Tabelle).

Kurzes, kräftiges Haar kann sich gut mit Gelen, Wachsen, Pasten, Cremes und Haarsprays (siehe Tabelle) in Form bringen und halten lassen.

Je länger die Haare sind, desto stärker kann der Wunsch des Nutzers sein, das Stylingprodukt vor dem zu Bett gehen aus dem Haar zu entfernen. Kurze Haare, die mit Wachsen, Pasten, Cremes, Gelen o.ä. gestylt wurden lassen sich zumeist schnell mit Wasser auswaschen und trocknen auch im Anschluss schnell (ggf. durch zur Hilfenahme eines Föhns). Längere Haare, die mit einem Stylingmousse in Form gebracht werden und/oder mit einem Haarspray fixiert werden, werden oftmals ausgebürstet. Zu diesem Zweck kann es hilfreich sein, wenn das angewendete fixierende Polymer leicht spröde und brüchig wird und sich z.B. durch eine Zurhilfenahme eines Kammes/einer Bürste entfernen lässt. Besagte Polymere wären bspw. PVP, PVP/VA, Amphomer, etc. (siehe Tabelle).

Nutzer mit afro-amerikanischem Haar können Produkte bevorzugen, die vor allem eines mit sich bringen: viel Pflege. Diese Zielgruppe kann das Gefühl haben, dass ihre Haare sehr trocken und spröde sind und viel Feuchtigkeit benötigen. Hier können insbesondere reichhaltige, cremige Texturen erwünscht sein, aber auch Ölsprays, die die Frisur des Nutzers gesund und gepflegt aussehen lassen. Konditionierende Polymere können helfen, das Haar in Form zu halten, ohne es steif und unflexibel wirken zu lassen. Reichhaltige Wachse und Emulsionen können den Haaren zusätzlich Pflege bereitstellen und können die Haare leicht beschweren, sodass die Haare nicht fliegen (Anti-Frizz). Was auf europäischem Haar also ein "no go" wäre, kann für afroamerikanisches Haar gar nicht pflegend/beschwerend/fettig genug sein.

Nutzer mit geschädigtem, z.B. blondiertem, Haar können mehr Pflege in ihren Produkten benötigen als Personen mit gesundem ("virgin") Haar.

In verschiedenen Ausführungsbeispielen können zusätzlich zu den chemischen Eigenschaften oder stattdessen physikalische Eigenschaften der empfohlenen und nicht zu empfehlenden Pflege- und Stylingprodukte (DOs und DON'Ts, z.B. Viskositäten, Verdampfungseigenschaften, Klebrigkeit) ausschlaggebend oder einflussreich bezüglich dessen sein, ob ein Produkt für einen gegebenen Haartyp als empfehlenswert betrachtet wird oder nicht.

Je länger das Haar, desto geringer sollte eine Klebrigkeit von Formulierungen sein. Gerade Nutzer(innen) mit langem Haar wünschen sich häufig ein Stylingprodukt, welches hält und stylt, allerdings nicht stark klebt. Viele von ihnen können ein natürliches Haargefühl und -aussehen bevorzugen.

Neben Mousses und leichten Sprays oder Emulsionen zum Trockenföhnen können langhaarige Frauen auch häufig Haarspray verwenden, um ihre Frisur zu fixieren. Auch hier kann ein nicht stark klebendes Produkt bevorzugt sein oder werden. Zusätzlich kann es allerdings wünschenswert sein, dass das Haarspray feinst verteilt auf dem Haar ankommt, um oben besagten Effekt (eine festbetonierte/unnatürlich aussehende Frisur) zu vermeiden.

Auch Nutzer mit Locken können Wert darauf legen, die Locken zu unterstützen und natürlich aussehen zu lassen, und ihren Look nicht zu zementieren. Je dicker und kürzer das Haar, desto höher darf die Produktviskosität sein, da diese oftmals schon bei Stylen hilft, das Haar in Form zu bringen. Hier können sich, wie vorab bereits beschrieben, Pasten, Cremes, Gele mit einer hohen Viskosität eignen.

In verschiedenen Ausführungsbeispielen kann zusätzlich zu den chemischen Eigenschaften und/oder den physikalischen Eigenschaften eine Konfektion (auch als Anwendungsform, Aufbringungsform usw. bezeichnet) der empfohlenen und nicht zu empfehlenden Pflege- und Stylingprodukte (DOs und DON'Ts, z.B. eher Spray als Gel) ausschlaggebend oder einflussreich bezüglich dessen sein, ob ein Produkt für einen gegebenen Haartyp als empfehlenswert betrachtet wird oder nicht. Dies ist teilweise oben beschrieben.

In verschiedenen Ausführungsbeispielen kann die ermittelte Empfehlung genutzt werden für eine Herstellung eines optimalen/personalisiertem Stylingprodukts, z.B. als ein Auftrag für eine Herstellung eines optimalen/personalisiertem Stylingprodukts.

Dies kann beispielsweise durch Aufrufen einer Internetseite eines Herstellers von optimalen/personalisierten Haarbehandlungsprodukten, wie beispielsweise Stylingprodukten, einzuleiten.

Bei dem optimalen/personalisierten Haarbehandlungsprodukt kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Mit Hilfe eines Produktkonfigurators kann beispielsweise die Auswahl chemisch und/oder physikalisch inkompatibler Inhaltsstoffe oder die Auswahl für den ermittelten Haarzustand ungeeigneter Inhaltsstoffe vermieden werden. Umgekehrt kann die Auswahl für den ermittelten Haarzustand geeigneter Inhaltsstoffe durch den Produktkonfigurator vorgegeben oder vorgeschlagen werden.

In verschiedenen alternativen Ausführungsbeispielen kann eine Nutzereingabe an einer Abgabevorrichtung zur optimierten Abgabe von Haarbehandlungsprodukt genutzt werden. Diese Abgabevorrichtung kann vorzugsweise bei einem Friseur oder an einem Verkaufspunkt ("point of sale", POS) von Haarbehandlungsmitteln vorhanden sein. Der Nutzer kann mithilfe einer digitalen Anzeige und eines Touchscreens seinen Haarzustand und ggf. die gewünschte Menge an Produkt auswählen. Hierzu kann eine gespeicherte Empfehlung bereits im Gerät einprogrammiert sein, beispielsweise in einer Datenbank abgelegt (d.h. gespeichert) sein. Derlei Empfehlungen können von Beipackzetteln von Haarcolorationen bekannt sein, auf welchen häufig vermerkt sein kann, dass z.B. Bei schulterlangem Haar zwei Colorationspackungen angewendet werden sollten. Mittels einer vorherigen individuellen Eingabe der Haarlänge des Nutzers kann so eine (Mengen- und) Produktempfehlung abgegeben werden. Diese kann vom Nutzer entweder bestätigt, erweitert oder verringert werden, wenn dieser beispielsweise selbst schon weiß, dass er tendenziell mehr/weniger Produkt anwendet, als auf Verpackungen normalerweise angegeben.

In verschiedenen Ausführungsbeispielen wird ein Verfahren zum kosmetischen Behandeln von Haaren bereitgestellt, welches eine richtige, d.h. zum Haarschädigungsgrad passende, Produktauswahl ermöglicht. Beispielsweise kann bei der Haarbehandlung ein exakt passendes Produkt für eine Haarcoloration, Blondierung, Dauerwelle, Haarpflege und/oder ein Haarstyling angesichts seines Haarschädigungsgrads verwendet werden.

In verschiedenen Ausführungsbeispielen kann ein Nutzer selbst seinen Haarschädigungsgrad und/oder weitere Haarzustandsparameter ermitteln, beispielsweise ohne aufwändige Mikroskopie durchzuführen und/oder Hintergrundwissen zu einer entsprechenden Auswertung zu haben. Somit kann es dem Nutzer ermöglicht werden, bei einem Ermitteln seines Haarzustands auf fachkundige Hilfe zu verzichten.

Hierin kann Bezug genommen werden auf "die Sensoren", beispielsweise hinsichtlich einer Datenübertragung zwischen den Sensoren und einer Datenverarbeitungsvorrichtung, einer Anordnung der Sensoren, usw. Dies ist so zu verstehen, dass die Sensoren eine Gesamtheit von in der Haarbehandlungsvorrichtung bzw. dem Haarbehandlungssystem angeordneten Sensoren aufweisen kann, z.B. eine Gesamtheit von Kamera/s, Temperatursensor/en, Mikrofon/en, usw., oder, sofern dies aus dem Kontext hervorgeht, einen Teil der genannten Sensoren.

In verschiedenen Ausführungsbeispielen kann das Haarbehandlungssystem eine elektronische Vorrichtung aufweisen, z.B. eine mobile elektronische Vorrichtung (auch als Mobile Device bezeichnet), z.B. ein Smartphone oder ein Tablet, oder z.B. eine sonstige Datenverarbeitungsvorrichtung (z.B. einen PC). In verschiedenen Ausführungsbeispielen kann das Haarbehandlungssystem ferner eine (ggf. weitere) externe Datenverarbeitungsvorrichtung, z.B. eine Cloud, nutzen für eine Signalauswertung, z.B. als eine Erweiterung der Signalauswertung.

Dafür können in verschiedenen Ausführungsbeispielen die mittels der Sensoren erfassten Signale mit in einer Datenbank hinterlegten Signalen (auch als Vergleichssignale, Vergleichsdaten, Referenzsignale oder Referenzdaten bezeichnet) verglichen werden. In verschiedenen Ausführungsbeispielen kann anhand dessen der Haarschädigungsgrad oder ein sonstiger Haarzustandsparameter eingeordnet werden, beispielsweise indem den Vergleichssignalen Haarschädigungsgrade zugeordnet sind, und der Haarschädigungsgrad bzw. der sonstige Haarzustandsparameter des dem gemessenen Signals ähnlichsten Vergleichssignals den gemessenen Haaren zugeordnet wird.

Die Referenzdaten, die z.B. als eine Datenbank bereitgestellt sein können, können in verschiedenen Ausführungsbeispielen empirisch (z.B. im Labor) gewonnen sein für Haare, deren Haarschädigungsgrad bekannt sein kann und. In verschiedenen Ausführungsbeispielen können ferner weitere Informationen über die Haare vorliegen, die als Grundlage für die Referenzspektren dienen, z.B. "viermal gebleichtes Haar - hoher Schädigungsgrad" oder "unbehandeltes Haar - keine Schädigung", und/oder für die Haare, die für das Ermitteln der Referenzspektren genutzt werden, kann eine Entwicklung eines Haarzustands, z.B. des Haarschädigungsgrads, bereitgestellt sein, z.B. mehrere Referenzspektren, von denen jedes nach einer anderen Haarbehandlungsstufe aufgenommen wurde, wobei die Haarbehandlung eine pflegende Haarbehandlung und/oder eine schädigende Haarbehandlung aufweisen kann. Ein Mittel (z.B. Produkt und/oder Inhaltsstoff), welches bei einer Behandlung genutzt wurde, kann außerdem mittels der Datenbank erfasst worden sein.

In verschiedenen Ausführungsbeispielen kann einem Nutzer auch die (z.B. der Datenbank entnommene) Zusatzinformation bereitgestellt werden, beispielsweise welchem Behandlungsgrad sein Haarzustand entspricht, und/oder wie sich sein Haarzustand voraussichtlich entwickeln wird, wenn er eine bestimmte Behandlung vornimmt, z.B. ein bestimmtes Mittel anwendet.

In verschiedenen Ausführungsbeispielen, z.B. bei Verwendung einer Cloud, kann die Datenbank alternativ zu einem Erzeugen im Labor mittels Nutzerdaten erzeugt werden (und beispielsweise fortlaufend ergänzt werden). In verschiedenen Ausführungsbeispielen kann die im Labor erzeugte Datenbank mittels Nutzerdaten, welche mittels der Cloud bereitgestellt werden können, ergänzt werden.

In verschiedenen Ausführungsbeispielen kann die Haarbehandlungsvorrichtung bereitgestellt sein als ein Zubehör, z.B. ein "kluges Zubehör" (auch als "smartes Zubehör" bezeichnet) für ein Smartphone (oder ein ähnliches Gerät wie z.B. ein Tablet, ein iPod o.ä.), welches mit dem Smartphone verbunden, z.B. daran angesteckt werden kann, womit ermöglicht werden kann, Verarbeitungsmöglichkeiten und/oder Sensoren des Smartphones zu nutzen. In verschiedenen Ausführungsbeispielen kann das Haarbehandlungssystem ein solches Zubehör aufweisen.

In verschiedenen Ausführungsbeispielen kann das Haarbehandlungssystem bereitgestellt sein als eine eigenständige Vorrichtung, welche beispielsweise eine eigene Vorrichtung zum Übertragen von Daten aufweisen kann und somit eine so genannte "Internet-of-Things (loT)"-Vorrichtung sein kann. Die eigenständige Haarbehandlungsvorrichtung kann die aufgezeichneten Daten (z.B. akustische Daten und ggf. Geschwindigkeitsdaten) beispielsweise mittels Bluetooth, WLAN (WiFi), NFC oder ähnlichem an eine externe Datenverarbeitungsvorrichtung übertragen, z.B. zu einer Cloud.

In verschiedenen Ausführungsbeispielen können die aufgezeichneten Daten, wie hierin an anderer Stelle beschrieben, mittels Softwarealgorithmen analysiert werden, um einen Haarzustandsparameter, z.B. einen Haarschädigungsgrad, zu ermitteln.

In verschiedenen Ausführungsbeispielen, beispielsweise wenn die Referenzdaten mittels der Cloud bereitgestellt werden, können diese einem Nutzer jederzeit zur Verfügung stehen, um als Referenzdaten für einen Vergleich genutzt zu werden.

In verschiedenen Ausführungsbeispielen können die mittels der Haarbehandlungsvorrichtung bzw. mittels des Haarbehandlungssystems erfassten Daten gespeichert werden, beispielsweise in einem in der Haarbehandlungsvorrichtung integrierten Speicher und/oder in der externen Datenverarbeitungsvorrichtung, z.B. der Cloud. Die gespeicherten Daten können so abgespeichert werden, dass zumindest dem Nutzer ermöglicht ist, diese Daten als seine Daten zu erkennen. Damit kann ein Vergleichen von Haarinformationen, die beispielsweise zu verschiedenen Zeitpunkten gewonnen wurden (z.B. vor und nach einer Behandlung) miteinander ermöglicht werden.

In verschiedenen Ausführungsbeispielen können die Haarbehandlungsvorrichtung und/oder das Haarbehandlungssystem eine Verbindung zum Übertragen von Daten aufweisen, beispielsweise zwischen einem Smartphone/Tablet, welches Teil des Haarbehandlungssystems sein kann, und einer Cloud, und/oder zwischen der Haarbehandlungsvorrichtung und einem Smartphone/Tablet, und/oder zwischen einer Haarbehandlungsvorrichtung und einer Cloud.

Für die Datenübertragung kann in verschiedenen Ausführungsbeispielen ein bekannter Datenübertragungsstandard genutzt werden, z.B. Bluetooth, WLAN (WiFi), NFC oder ähnliches. In verschiedenen Ausführungsbeispielen kann die Haarbehandlungsvorrichtung bzw. das Haarbehandlungssystem eine entsprechende Datenübertragungsvorrichtung zum Senden und/oder Empfangen der Daten aufweisen.

In verschiedenen Ausführungsbeispielen können mittels der Haarbehandlungsvorrichtung erhobene Daten (d.h. der ermittelte mindestens eine Sensorwert und/oder basierend darauf ermittelte Daten und/oder Empfehlungen und/oder ein Steuerungs- bzw. Regelungsparameter) bereitgestellt werden.

In verschiedenen Ausführungsbeispielen kann die Analyse durch die Haarbehandlungsvorrichtung selbst, beispielsweise mittels der Schaltkreisvorrichtung, erfolgen, und ein Analyseergebnis kann zum Bereitstellen des Analyseergebnisses an eine Anzeigevorrichtung übertragen werden, beispielsweise an ein Display, einen Lautsprecher, ein Smartphone oder ähnliches.

In verschiedenen Ausführungsbeispielen können die Daten übertragen werden auf/an eine externe Datenverarbeitungsvorrichtung (auch als externe Plattform bezeichnet), z.B. auf ein Smartphone mit App, an eine Cloud, usw. Nach der Datenübertragung auf die externe Datenverarbeitungsvorrichtung kann mittels dieser die Untersuchung der Daten ausgeführt werden, beispielsweise zum Ermitteln eines Steuerungs- bzw. Regelungsparameters und/oder einer Empfehlung.

In verschiedenen Ausführungsbeispielen kann eine Abstimmung der kosmetischen Behandlung mit individualisierten Konsumentendaten einen iterativen kosmetischen Behandlungszyklus ermöglichen, ein Ergebnis der kosmetischen Behandlung verbessern und/oder eine Motivation des Nutzers, die Behandlung fortzusetzen, erhöhen.

In verschiedenen Ausführungsbeispielen können einem Nutzer Informationen über einen Status des Haares (auch als Haarzustand bezeichnet) bereitgestellt werden, welche ferner genutzt werden können, um eine individuelle, auf den Haarzustand des Nutzers abgestimmte Steuerung bzw. Regelung der Haarbehandlungsvorrichtung und/oder eine Empfehlung zu ermitteln, beispielsweise eine Produktempfehlung (z.B. für ein Haarpflege- und/oder ein Haarstylingprodukt und/oder ein Haarfärbeprodukt) und/oder eine Pflegeempfehlung, z.B. eine Pflegeempfehlung, welche die Haare des Nutzers betrifft.

In verschiedenen Ausführungsbeispielen kann ein Haarfärbeprodukt von den zu verwendenden Haarbehandlungsmitteln ausgenommen sein.

In verschiedenen Ausführungsbeispielen kann der Steuerungs- bzw. Regelungsparameter bzw. die Empfehlung direkt mittels der Haarbehandlungsvorrichtung ermittelt sein oder werden, d.h. die elektronische Schaltkreisvorrichtung kann eingerichtet sein, den Steuerungs-/Regelungsparameter bzw. die Empfehlung selbst (auch als direkt bezeichnet) zu ermitteln. Beispielsweise kann die elektronische Schaltkreisvorrichtung eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und entweder dem Nutzer direkt bereitzustellen, oder um die Sensordaten zu verwenden, um die Empfehlung bereitzustellen. Beispielsweise können die Sensordaten mit einer Datenbank verglichen werden, welche beispielsweise empirisch gewonnen worden sein kann. In der Datenbank können einer Mehrzahl von Sensordaten Empfehlungen zugeordnet sein.

In verschiedenen Ausführungsbeispielen kann die elektronische Schaltkreisvorrichtung eingerichtet sein, den Steuerungs- bzw. Regelungsparameter bzw. die Empfehlung, z.B. Produkt- oder Behandlungsempfehlung, indirekt zu ermitteln. Beispielsweise kann die elektronische Schaltkreisvorrichtung (z.B. zusätzlich zu einem Speicher und einem Prozessor, z.B. einem Mikroprozessor) mit einer Datenübertragungsvorrichtung ausgerüstet sein, welche eingerichtet sein kann, die von der elektronische Schaltkreisvorrichtung empfangenen Sensordaten an eine externe Datenverarbeitungsvorrichtung, z.B. an einen Computer, z.B. eine Cloud, zu übermitteln, mittels derer, beispielsweise wie oben für das Ermitteln der Steuerungs- bzw. Regelungsparameter bzw. der Empfehlung mittels der elektronischen Schaltkreisvorrichtung beschrieben, der Steuerungs- bzw. Regelungsparameter bzw. die Empfehlung ermittelt werden kann, um den Steuerungs- bzw. Regelungsparameter bzw. die Empfehlung bereitzustellen, beispielsweise mittels Übertragens an eine Anzeigevorrichtung und/oder mittels Übertragens der Empfehlung zurück an die elektronische Schaltkreisvorrichtung (z.B. mittels der Datenübertragungsvorrichtung). In verschiedenen Ausführungsbeispielen kann die Datenübertragung mehrstufig erfolgen, beispielsweise indem die Sensordaten von der Schaltkreisvorrichtung zunächst an die Anzeigevorrichtung (z.B. ein Smartphone, ein Tablet o.ä.) übermittelt werden, und die Anzeigevorrichtung die Sensordaten an die externe Datenverarbeitungsvorrichtung (z.B. die Cloud) überträgt.

In verschiedenen Ausführungsbeispielen kann das Bereitstellen der Steuerungs- bzw. Regelungsparameter bzw. der Empfehlung ein Einstellen von Steuerungs- bzw. Regelungsparametern bei der zu steuernden bzw. zu regelnden Vorrichtung (z.B. beheizbarer Teil der Vorrichtung, Pumpe einer Abgabevorrichtung o.ä.) und/oder ein Bereitstellen mittels eines Übertragens der Empfehlung an eine Anzeigevorrichtung und Anzeigen der Empfehlung aufweisen.

Das Übertragen kann in verschiedenen Ausführungsbeispielen mittels einer drahtlosen Übertragungsvorrichtung erfolgen. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN (WiFi), ZigBee, NFC, Wibree, Threald, WiMAX oder ähnlichem ermöglicht.

In verschiedenen Ausführungsbeispielen kann die Anzeigevorrichtung einen Computerbildschirm, ein Smartphone, ein Tablet, ein iPad, einen Smart Mirror, eine Smart Watch, einen Laptop oder ähnliches aufweisen.

In verschiedenen Ausführungsbeispielen wird eine Haarbehandlungsvorrichtung bereitgestellt. Die Haarbehandlungsvorrichtung kann einen Vorrichtungskörper aufweisen, mindestens einen im oder am Vorrichtungskörper angeordneten Sensor zum Erfassen eines Haarzustandsparameters und eine im oder am Vorrichtungskörper angeordnete elektronische Schaltkreisvorrichtung, wobei die elektronische Schaltkreisvorrichtung mit dem mindestens einen Sensor zum Erfassen eines Haarzustandsparameters gekoppelt sein kann zum Empfangen des erfassten Haarzustandsparameters, und wobei die elektronische Schaltkreisvorrichtung ferner eingerichtet sein kann, basierend auf dem empfangenen erfassten Haarzustandsparameter mindestens einen Haar-Behandlungsparameter zu steuern und/oder mindestens ein Haar-Behandlungsmittel zu dosieren und/oder eine Haarbehandlungsempfehlung bereitzustellen.

In verschiedenen Ausführungsbeispielen kann die Haarbehandlungsvorrichtung ein Glätteisen aufweisen.

In verschiedenen Ausführungsbeispielen kann der gesteuerte Haarbehandlungsparameter eine Temperatur der Haarbehandlungsvorrichtung aufweisen.

In verschiedenen Ausführungsbeispielen kann der mindestens eine Sensor zum Erfassen eines Haarzustandsparameters eingerichtet sein, während des Erfassens des mindestens einen Haarzustandsparameters mit den Haaren des Nutzers in Berührung gebracht zu werden.

In verschiedenen Ausführungsbeispielen kann die elektronische Schaltkreisvorrichtung ferner eingerichtet sein, basierend auf dem empfangenen Sensorwert eine Haarzustandsinformation und/oder eine Empfehlung zu ermitteln und dem Nutzer bereitzustellen.

In verschiedenen Ausführungsbeispielen kann die Empfehlung mindestens eine Empfehlung ausgewählt aus der Gruppe bestehend aus Haarpflegeproduktempfehlungen, Haarstylingproduktempfehlungen und Haarbehandlungsempfehlung aufweisen.

In verschiedenen Ausführungsbeispielen kann der mindestens eine Sensor im Vorrichtungskörper versiegelt angeordnet sein.

In verschiedenen Ausführungsbeispielen kann die elektronische Schaltkreisvorrichtung eine kabellose Datenaustauschvorrichtung aufweisen.

In verschiedenen Ausführungsbeispielen kann der Sensor zum Erfassen eines Haarzustandsparameters mindestens einen Sensor aufweisen gewählt aus einer Gruppe von Sensoren, die Gruppe von Sensoren aufweisend: eine Kamera zum Aufnehmen eines digitalen Bildes der Haare, einen Temperatursensor, einen Feuchtigkeitssensor, ein Mikrofon und einen Zugkraftmesser.

In verschiedenen Ausführungsbeispielen wird ein Haarbehandlungssystem bereitgestellt. Das Haarbehandlungssystem kann eine Haarbehandlungsvorrichtung gemäß verschiedenen Ausführungsbeispielen und
eine Anzeigevorrichtung aufweisen, wobei die mindestens eine Haarbehandlungsvorrichtung eingerichtet sein kann, der Anzeigevorrichtung die Haarzustandsinformation und/oder die Empfehlung mittels der Datenaustauschvorrichtung zu übermitteln.

In verschiedenen Ausführungsbeispielen kann die Anzeigevorrichtung einen Computerbildschirm, ein Smartphone, ein Tablet, ein iPad, einen Smart Mirror, eine Smart Watch oder einen Laptop aufweisen.

In verschiedenen Ausführungsbeispielen wird ein Haarbehandlungssystem bereitgestellt. Das Haarbehandlungssystem kann einen Vorrichtungskörper aufweisen, mindestens einen im oder am Vorrichtungskörper angeordneten Sensor zum Erfassen eines Haarzustandsparameters, eine im oder am Vorrichtungskörper angeordnete elektronische Schaltkreisvorrichtung mit einer kabellosen Datenaustauschvorrichtung und eine Datenverarbeitungsvorrichtung, wobei die elektronische Schaltkreisvorrichtung mit dem mindestens einen Sensor gekoppelt sein kann zum Empfangen des erfassten Haarzustandsparameters, wobei die elektronische Schaltkreisvorrichtung ferner eingerichtet sein kann, mittels der kabellosen Datenaustauschvorrichtung der Datenverarbeitungsvorrichtung den empfangenen Haarzustandsparameter zu übermitteln, wobei die Datenverarbeitungsvorrichtung eingerichtet sein kann, basierend auf dem empfangenen erfassten Haarzustandsparameter, eine Steuerung eines Haar-Behandlungsparameters und/oder eine Dosierung eines Haar-Behandlungsmittels zu ermitteln und der elektronischen Schaltkreisvorrichtung oder einer in einer Behandlungsvorrichtung angeordneten weiteren elektronischen Schaltkreisvorrichtung mittels der kabellosen Datenaustauschvorrichtung zu übertragen, und
wobei die elektronische Schaltkreisvorrichtung oder die weitere elektronische Schaltkreisvorrichtung ferner eingerichtet sein kann, den mindestens einen Haar-Behandlungsparameter zu steuern und/oder das mindestens eine Haar-Behandlungsmittel zu dosieren.

In verschiedenen Ausführungsbeispielen kann die Datenverarbeitungsvorrichtung ein Smartphone, ein iPad oder ein Tablet aufweisen.

In verschiedenen Ausführungsbeispielen wird ein Verfahren zum kosmetischen Behandeln von Haaren eines Nutzers bereitgestellt. Das Verfahren kann aufweisen während eines Behandelns und/oder vor einem Behandeln der Haare mittels einer Haarbehandlungsvorrichtung gemäß verschiedenen Ausführungsbeispielen oder mittels eines Haarbehandlungssystems gemäß verschiedenen Ausführungsbeispielen Erfassen eines Haarzustandsparameters mittels des mindestens einen Sensors und
Steuern mindestens eines Haarbehandlungsparameters und/oder Dosieren mindestens eines Haar-Behandlungsmittels und/oder Empfehlen mindestens einer Haarbehandlung basierend auf dem mindestens einen Haazustandsparameter.

In verschiedenen Ausführungsbeispielen kann der Haarbehandlungsparameter eine Temperatur der Haarbehandlungsvorrichtung aufweisen.

In verschiedenen Ausführungsbeispielen kann die Haarbehandlungsvorrichtung ein Glätteisen aufweisen.

In verschiedenen Ausführungsbeispielen kann das Verfahren ferner ein Übermitteln des erfassten Haarzustandsparameters an die Datenverarbeitungsvorrichtung aufweisen, und ein Empfangen der von der externen Datenverarbeitungsvorrichtung bereitgestellten Information, wobei das Ermitteln des mindestens einen Haarbehandlungsparameters und/oder der Haar-Behandlungsmitteldosierung basierend auf dem mindestens einen Haarzustandsparameters mittels der Datenverarbeitungsvorrichtung erfolgen kann.

In verschiedenen Ausführungsbeispielen kann die externe Datenverarbeitungsvorrichtung eine Cloud aufweisen oder sein.

Ausführungsbeispiele der Erfindung sind in den Figuren dargestellt und werden im Folgenden näher erläutert.

Es zeigen
- Figur 1: eine schematische Darstellung einer Haarbehandlungsvorrichtung gemäß verschiedenen Ausführungsbeispielen;
- Figur 2A: bis Figur 2C jeweils eine schematische Darstellung eines Haarbehandlungssystems gemäß verschiedenen Ausführungsbeispielen;
- Figur 3: eine schematische Darstellung einer Anwendung einer Haarbehandlungsvorrichtung gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

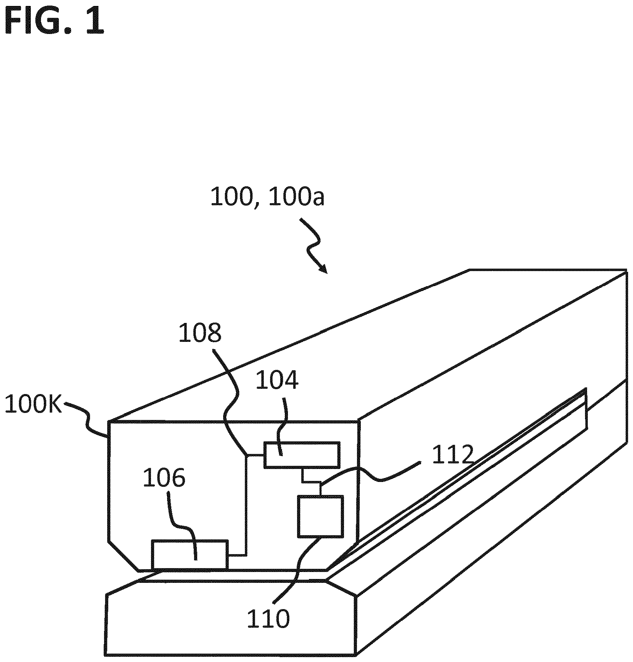

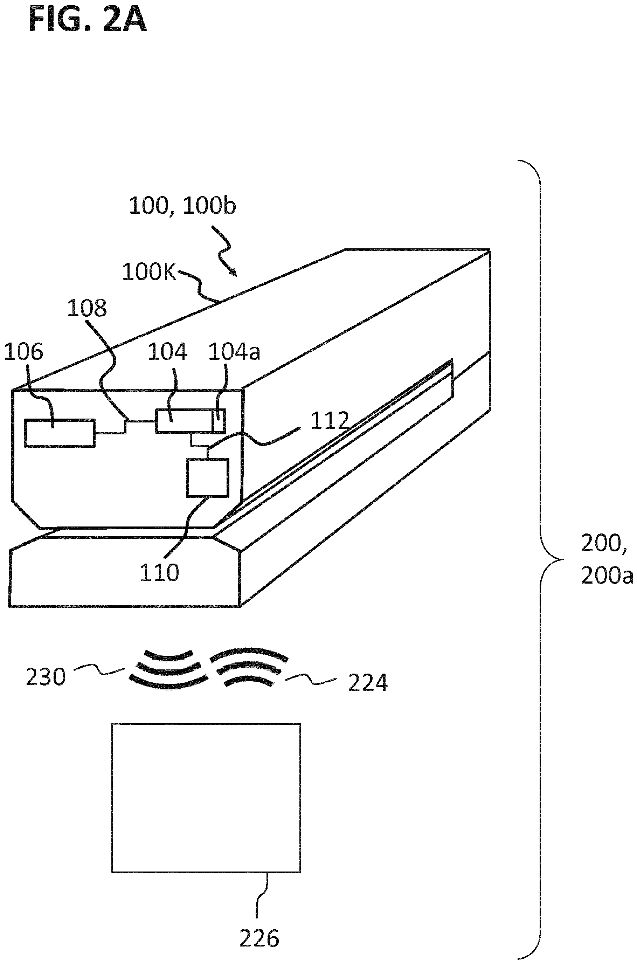

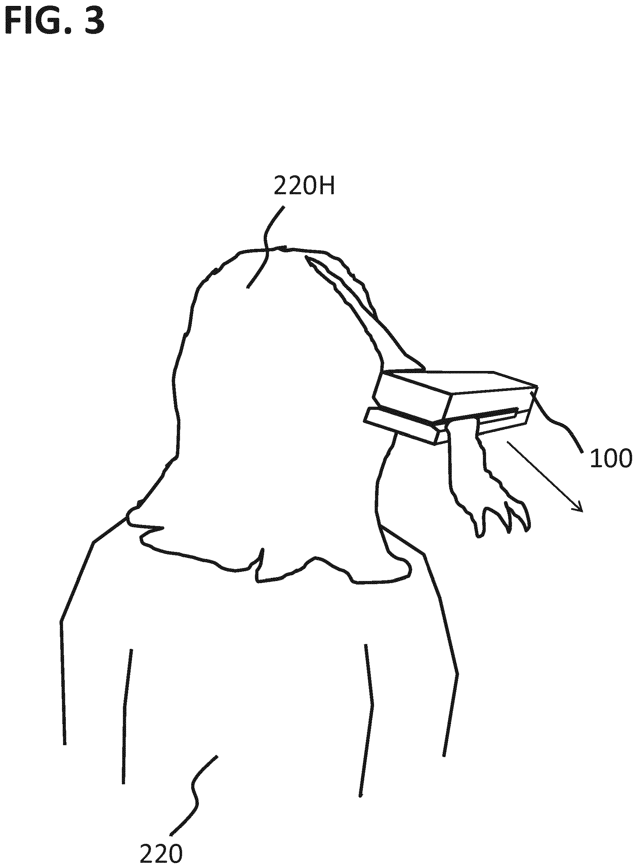

FIG. 1 zeigt eine schematische Darstellung einer Haarbehandlungsvorrichtung 100 gemäß verschiedenen Ausführungsbeispielen, FIG. 2A bis FIG. 2C zeigen jeweils eine schematische Darstellung eines Haarbehandlungssystems 200 gemäß verschiedenen Ausführungsbeispielen, FIG. 3 zeigt eine schematische Darstellung einer Anwendung einer Haarbehandlungsvorrichtung 100 gemäß verschiedenen Ausführungsbeispielen.

In verschiedenen Ausführungsbeispielen wird ein Haarbehandlungsvorrichtung 100 (verschiedene Ausführungsbeispiele sind als 100a, 100b usw. gekennzeichnet) bereitgestellt.

Auch wenn in den Figuren die Haarbehandlungsvorrichtung 100 schematisch als Glätteisen dargestellt ist, ist zu verstehen, dass die Haarbehandlungsvorrichtung auch von anderer Art sein kann, beispielsweise wie oben beschrieben. Beispielsweise kann die Haarbehandlungsvorrichtung einen Lockenstab, einen Kamm, eine Bürste, eine Trockenhaube oder ähnliches aufweisen. Sofern anwendbar ist zu verstehen, dass die Ausführungen im Zusammenhang mit den Figuren auch für solche Arten von Haarbehandlungsvorrichtungen gelten sollen.

In verschiedenen Ausführungsbeispielen kann die Haarbehandlungsvorrichtung 100 einen Vorrichtungskörper 100K aufweisen.

Der Vorrichtungskörper 100K kann aus einem festen Material, z.B. Kunststoff oder Metall, gebildet sein oder ein solches Material aufweisen. Beispielsweise kann der Vorrichtungskörper 100K aus einem Material gebildet sein oder ein Material aufweisen, welches üblicherweise für ein Glätteisen, einen Kamm, eine Bürste oder ähnliches verwendet wird.

In verschiedenen Ausführungsbeispielen, insbesondere wenn die Haarbehandlungsvorrichtung 100, 100a eine Vorrichtung zur semipermanenten oder temporären Haarumformung aufweist, z.B. das Glätteisen, den Lockenstab oder die Trockenhaube, kann der Haarbehandlungsvorrichtung 100, 100a ferner eine steuer- bzw. regelbare Heizvorrichtung aufweisen (nicht dargestellt), welche beispielsweise in den Vorrichtungskörper 100K integriert sein kann.

In verschiedenen Ausführungsbeispielen kann die Haarbehandlungsvorrichtung 100 mindestens zwei im oder am Vorrichtungskörper angeordnete Sensoren 106 aufweisen. Ein Sensor dient zum Erfassen mindestens eines Haarzustandsparameters, und umfasst beispielsweise eines Haarzustandsparameters wie oben ausgeführt, z.B. einen Haarfeuchtigkeitssensor, einen Haarschädigungsgradsensor, einen Haardickesensor, einen Grauanteilsensor, einen Haardichtesensor, einen Lockigkeitssensor oder ähnliches. Der zweite Sensor dient zum Erfassen von Bewegungen und Ortsveränderungen und umfasst insbesondere ein Gyroskop.

In verschiedenen Ausführungsbeispielen kann der mindestens eine Sensor zum Erfassen eines Haarzustandsparameters eingerichtet sein, mehr als einen Parameter zu erfassen, beispielsweise kann der Sensor ein NIR-Spektrometer aufweisen, welches eingerichtet sein kann, sowohl Parameter zum Ermitteln der Haarfeuchtigkeit als auch Parameter zum Ermitteln des Haarschädigkeitsgrads zu erfassen, und/oder der Sensor zum Erfassen eines Haarzustandsparameters kann eine Kamera aufweisen, welche eingerichtet sein kann, sowohl Parameter zum Ermitteln der Lockigkeit als auch Parameter zum Ermitteln des Grauanteils zu ermitteln.

In verschiedenen Ausführungsbeispielen können die mindestens zwei Sensoren 106 im Vorrichtungskörper 100K eingebaut sein, beispielsweise versiegelt eingebaut sein. Damit kann ermöglicht sein, dass die Haarbehandlungsvorrichtung 100 unempfindlich ist gegen Feuchtigkeit und Schmutz. Beispielsweise kann mittels des Versiegelns erreicht werden, dass die Haarbehandlungsvorrichtung gereinigt werden kann, ohne die Sensoren 106 oder eine andere Vorrichtung zu beschädigen. Die Sensoren 106 können beispielsweise bei einem Spritzgießen des Vorrichtungskörpers 100K mit eingegossen werden.

In verschiedenen Ausführungsbeispielen, z.B. wenn die mindestens zwei Sensoren 106 einen optischen Sensor aufweisen, kann der Vorrichtungskörper 100K zwischen dem optischen Sensor und einer Oberfläche des Vorrichtungskörpers 100K transparent sein. In verschiedenen Ausführungsbeispielen können, sofern das zweckdienlich ist, die Sensoren 106 derart im Vorrichtungskörper 100 angeordnet sein, dass sie dem Haar 220H des Nutzers 220 bei einer üblichen Anordnung der Haare an oder in der Haarbehandlungsvorrichtung 100 zugewandt sind oder mit dem Haar 220H in Kontakt sind.

In verschiedenen Ausführungsbeispielen kann die Haarbehandlungsvorrichtung eine im oder am Vorrichtungskörper 100K angeordnete elektronische Schaltkreisvorrichtung 104 aufweisen.

Die elektronische Schaltkreisvorrichtung 104 kann in verschiedenen Ausführungsbeispielen mit den mindestens zwei Sensoren 106 gekoppelt sein, beispielsweise mittels einer Verbindung 108, zum Empfangen des erfassten Sensorwerts. Die Schaltkreisvorrichtung 104 kann eine eigene Kopplung zu jedem der Sensoren 106 aufweisen. Die Kopplung kann in verschiedenen Ausführungsbeispielen eine elektrisch leitende Verbindung, eine (Glas-)faserverbindung und/oder eine kabellose Verbindung aufweisen oder sein. Die elektronische Schaltkreisvorrichtung 104 kann zum Empfangen der mindestens zwei Sensorwerte von den mindestens zwei Sensoren 106 eingerichtet sein.

In verschiedenen Ausführungsbeispielen kann die elektronische Schaltkreisvorrichtung 104 eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Daten von den Sensoren 106 zu empfangen und entweder direkt einen Haarbehandlungsparameter zu steuern oder zu regeln, oder um die Sensordaten zu verwenden, um dem Nutzer 220 eine Empfehlung bereitzustellen.

Die elektronische Schaltkreisvorrichtung 104 kann in verschiedenen Ausführungsbeispielen eingerichtet sein, basierend auf den empfangenen Daten von den Sensoren 106 mindestens eine Empfehlung zu ermitteln und dem Nutzer 220 bereitzustellen.

In verschiedenen Ausführungsbeispielen kann die Haarbehandlungsvorrichtung ferner einen steuer- bzw. regelbaren Aktor 110 aufweisen. Der Aktor 110 kann eingerichtet sein, einen Haarbehandlungsparameter zu beeinflussen. Eine Steuerung bzw. Regelung des Aktors 110 kann in verschiedenen Ausführungsbeispielen mittels der elektronischen Schaltkreisvorrichtung 104 erfolgen. Der Aktor 110 kann in verschiedenen Ausführungsbeispielen mittels einer Verbindung 112 mit der elektronischen Schaltkreisvorrichtung 104 verbunden sein. Die Verbindung 112 kann in verschiedenen Ausführungsbeispielen eine elektrisch leitende Verbindung, eine (Glas-)faserverbindung und/oder eine kabellose Verbindung aufweisen oder sein.

Der Aktor 110 kann in verschiedenen Ausführungsbeispielen eine Temperatursteuerung bzw. - regelung eines beheizbaren Teils der Haarbehandlungsvorrichtung 100 aufweisen. In verschiedenen Ausführungsbeispielen, z.B. in einem Fall, in welchem die mindestens zwei Sensoren 106 unter anderem genutzt werden, um einen Haarschädigungsgrad, eine Lockigkeit oder ähnliches zu ermitteln, kann die Haarbehandlungsvorrichtung 100 eingerichtet sein, den Aktor 110 zu steuern, z.B. eine dem Haarschädigungsgrad, der Lockigkeit o.ä. entsprechende Temperatur für die Haarbehandlung, z.B. einen Glättvorgang oder einen Lockungsvorgang, einzustellen.

In verschiedenen Ausführungsbeispielen, z.B. in einem Fall, in welchem die mindestens zwei Sensoren 106 z.B. zusätzlich genutzt werden, um eine Haartemperatur zu ermitteln, z.B. an Positionen vor und hinter der Heizvorrichtung, kann die Haarbehandlungsvorrichtung 100 eingerichtet sein, den Aktor 110 zu regeln, z.B. eine dem Haarschädigungsgrad, der Lockigkeit o.ä. entsprechende Temperatur für die Haarbehandlung, z.B. einen Glättvorgang oder einen Lockungsvorgang, einzustellen und anhand der erfassten Temperatur (z.B. insbesondere der Temperatur hinter der Heizvorrichtung, d.h. nach der Hitzebehandlung) nachzuregeln.

In verschiedenen Ausführungsbeispielen kann die Haarbehandlungsvorrichtung 100 eingerichtet sein, den Aktor 110 allein aufgrund von Temperatursensordaten zu regeln.

In verschiedenen Ausführungsbeispielen, z.B. im Fall eines Glätteisens oder eines Lockenstabs, kann der Aktor 110 eingerichtet sein, auf eine relativ hohe Temperatur, z.B. in eienm Bereich von etwa 200°C bis etwa 230°C, eingestellt zu werden, wenn ermittelt wird, dass das Haar 220H des Nutzers 220 beispielsweise ungeschädigt und/oder dick ist, und auf eine relativ niedrige Temperatur eingestellt zu werden, z.B. in einem Bereich von etwa 150°C bis etwa 180°C, wenn ermittelt wird, dass das Haar 220H des Nutzers 220 beispielsweise vorgeschädigt und/oder dünn ist.

In verschiedenen Ausführungsbeispielen kann dem Nutzer 220 außerdem eine Haarbehandlungsempfehlung bereitgestellt werden, z.B. im Fall des Glätteisens oder Lockenstabs eine Empfehlung "jede Haarsträhne für maximal fünf Sekunden behandeln" oder ähnliches.

In verschiedenen Ausführungsbeispielen kann der Aktor 110, beispielsweise alternativ oder zusätzlich zur Temperatursteuerung bzw. -regelung, eine steuer- bzw. regelbare Abgabevorrichtung (auch als Dosiervorrichtung bezeichnet) aufweisen, welche eingerichtet sein kann, ein Haarbehandlungsmittel basierend auf den erfassten Sensordaten zu dosieren.

Wie oben ausgeführt, kann die Abgabevorrichtung mindestens eine Pumpe und/oder mindestens ein Ventil aufweisen, welches derart steuer- bzw. regelbar sein kann, dass ein Volumen bzw. eine Menge des Haarbehandlungsmittels dosiert werden kann. Das Dosieren kann in verschiedenen Ausführungsbeispielen in Abhängigkeit von einer Position der Haarbehandlungsvorrichtung 100 am Haar 220H erfolgen, z.B. kann bei einem Ermitteln, dass die Haarbehandlungsvorrichtung 100 sich am Haaransatz befindet, eine andere Menge des Haarbehandlungsmittels abgegeben werden als bei einem Ermitteln, dass die Haarbehandlungsvorrichtung 100 sich an den Haarspitzen befindet. Beispielsweise können die Haarspitzen eine größere Menge eines Haarpflegemittels erfordern als der Haaransatz, während es bei einem Haarfärbemittel umgekehrt sein kann.

In verschiedenen Ausführungsbeispielen kann, wie in FIG. 2A dargestellt ist, ein Haarbehandlungssystem 200, 200a bereitgestellt sein.

Das Haarbehandlungssystem 200, 200a kann in verschiedenen Ausführungsbeispielen eine Haarbehandlungsvorrichtung 100, 100b aufweisen, welche in wesentlichen Teilen ähnlich oder identisch der Haarbehandlungsvorrichtung 100a sein kann.

Die Haarbehandlungsvorrichtung 100, 100b kann in verschiedenen Ausführungsbeispielen ferner eine Datenaustauschvorrichtung 104a aufweisen, welche eingerichtet sein kann, Daten kabellos zu übertragen und zu empfangen.

Das Haarbehandlungssystem 200, 200a kann in verschiedenen Ausführungsbeispielen ferner eine externe Datenverarbeitungsvorrichtung 226 aufweisen, welche z.B. Teil eines Smartphones, eines Tablets oder ähnliches sein kann, oder beispielsweise eine Cloud.

Die Datenverarbeitungsvorrichtung 226 kann, wie oben beschrieben, eingerichtet sein, die erfassten Sensordaten von der Datenverarbeitungsvorrichtung 100b zu empfangen, die Steuerungs- bzw. Regelungsparameter und/oder die mindestens eine Empfehlung zu ermitteln und/oder die Geschwindigkeit zu ermitteln und diese der Haarbehandlungsvorrichtung 100b zu übermitteln. Das heißt, die Haarbehandlungsvorrichtung 200b kann, wie oben beschrieben, eingerichtet sein, die Steuerungs- bzw. Regelungsparameter und/oder die mindestens eine Empfehlung und/oder die Geschwindigkeit indirekt zu ermitteln.

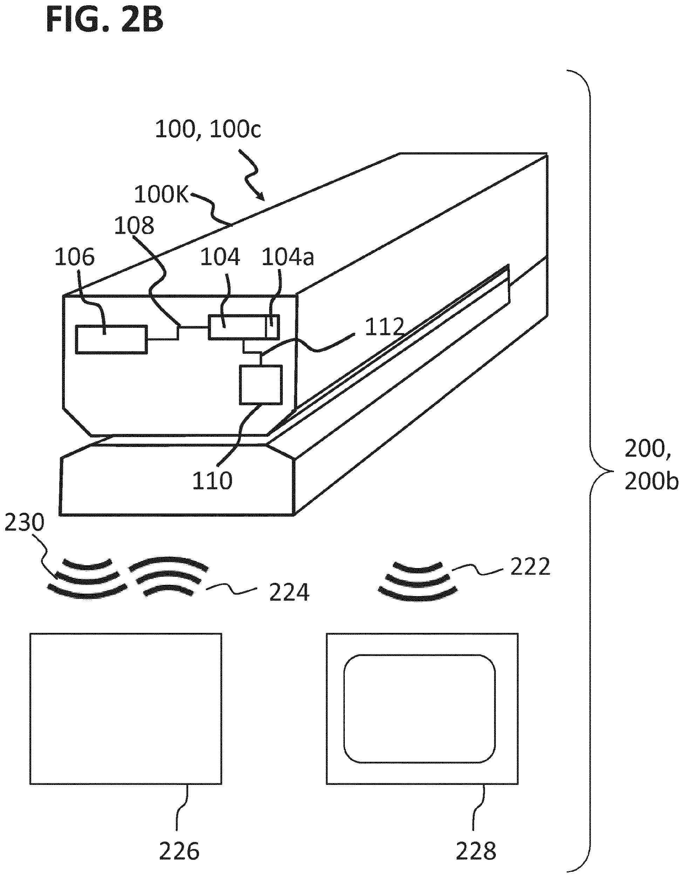

In verschiedenen Ausführungsbeispielen kann, wie in FIG. 2B dargestellt ist, ein Haarbehandlungssystem 200, 200b bereitgestellt sein.

Das Haarbehandlungssystem 200, 200b kann in verschiedenen Ausführungsbeispielen eine Haarbehandlungsvorrichtung 100, 100c aufweisen, welche in wesentlichen Teilen ähnlich oder identisch der Haarbehandlungsvorrichtung 100a und/oder der Haarbehandlungsvorrichtung 100b sein kann.

Die Datenaustauschvorrichtung 104a der Haarbehandlungsvorrichtung 100, 100c kann in verschiedenen Ausführungsbeispielen eingerichtet sein, Daten kabellos zu übertragen und zu empfangen.

Das Haarbehandlungssystem 200, 200b kann in verschiedenen Ausführungsbeispielen ferner eine Anzeigevorrichtung 228 aufweisen.

Die Datenverarbeitungsvorrichtung 226 kann, wie oben beschrieben, eingerichtet sein, die erfassten Sensordaten von der Datenverarbeitungsvorrichtung 100c zu empfangen, die Steuerungs- bzw. Regelungsparameter und/oder die mindestens eine Empfehlung und/oder die Geschwindigkeit zu ermitteln und diese der Haarbehandlungsvorrichtung 100c zu übermitteln. Das heißt, die Haarbehandlungsvorrichtung kann, wie oben beschrieben, eingerichtet sein, die Steuerungs- bzw. Regelungsparameter und/oder die mindestens eine Empfehlung und/oder die Geschwindigkeit indirekt zu ermitteln.

Die Anzeigevorrichtung 228 kann in verschiedenen Ausführungsbeispielen eingerichtet sein, Daten von der Haarbehandlungsvorrichtung 100c zu empfangen, beispielsweise eine mittels der elektronischen Schaltkreisvorrichtung 104 und/oder der externen Datenverarbeitungsvorrichtung 226 ermittelte Empfehlung, z.B. eine Haarbehandlungsempfehlung und/oder ein empfohlenes Haarpflegemittel. Die Anzeigevorrichtung 228 kann auch Daten hinsichtlich der räumlichen Lage der Haarbehandlungsvorrichtung 100c und/oder Daten hinsichtlich bereits behandelter Haarbereiche von der Haarbehandlungsvorrichtung 100c empfangen.

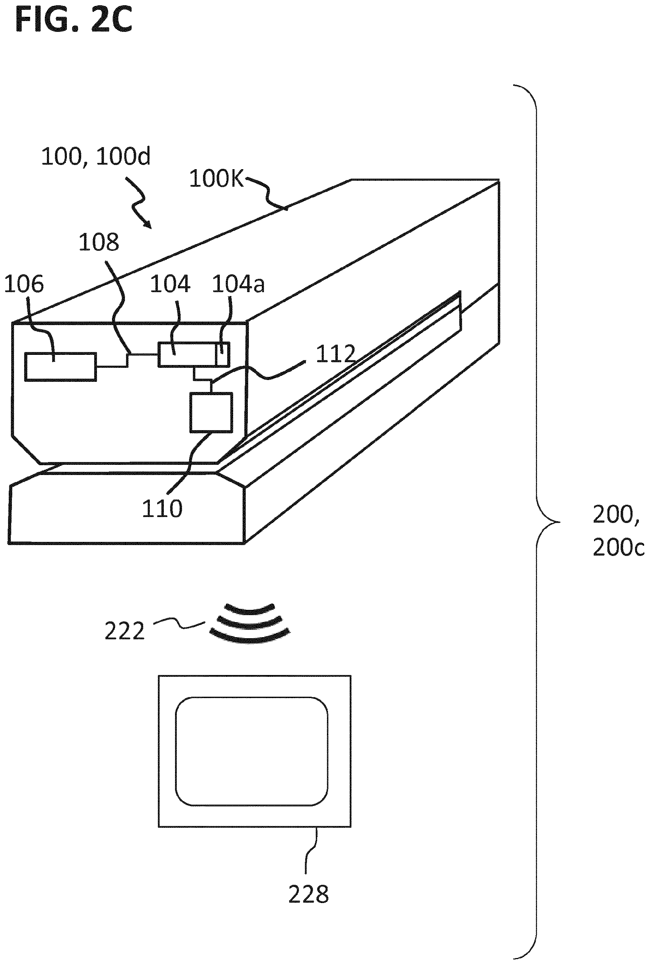

In verschiedenen Ausführungsbeispielen kann, wie in FIG. 2C dargestellt ist, ein Haarbehandlungssystem 200, 200c bereitgestellt sein.

Das Haarbehandlungssystem 200, 200c kann in verschiedenen Ausführungsbeispielen eine Haarbehandlungsvorrichtung 100, 100d aufweisen, welche in wesentlichen Teilen ähnlich oder identisch der Haarbehandlungsvorrichtung 100a und/oder der Haarbehandlungsvorrichtung 100b und/oder der Haarbehandlungsvorrichtung 100c sein kann.

Die Datenaustauschvorrichtung 104a der Haarbehandlungsvorrichtung 100, 100c kann in verschiedenen Ausführungsbeispielen eingerichtet sein, Daten kabellos zu übertragen und zu empfangen.

Das Haarbehandlungssystem 200, 200c kann in verschiedenen Ausführungsbeispielen eine Anzeigevorrichtung 228 aufweisen.

Die Anzeigevorrichtung 228 kann in verschiedenen Ausführungsbeispielen eingerichtet sein, Daten von der Haarbehandlungsvorrichtung 100d zu empfangen, beispielsweise eine mittels der elektronischen Schaltkreisvorrichtung 104 ermittelte Empfehlung, z.B. eine Haarbehandlungsempfehlung und/oder ein empfohlenes Haarpflegemittel oder mittels der elektronischen Schaltkreisvorrichtung 104 ermittelte Daten hinsichtlich der räumlichen Lage der Haarbehandlungsvorrichtung 100d.

In verschiedenen Ausführungsbeispielen kann zum Ermitteln eines Haarzustands, z.B. eines Haarschädigungsgrads o.ä., wie oben beschrieben eine Datenbank genutzt werden, in welcher den Sensordaten oder ggf. Kombinationen von Sensordaten, die Haarzustände (z.B. Haarschädigungsgrade) zugeordnet sein können. Die Datenbank kann vorab mittels Versuchen erstellt worden sein, und/oder kann fortwährend, z.B. bei Nutzung einer Cloud, mittels Nutzerdaten erstellt bzw. ergänzt werden.

In verschiedenen Ausführungsbeispielen kann die Datenbank ferner eine Produktempfehlung auf Basis des Haarzustands, z.B. des Haarschädigungsgrads, bereitstellen.

Wie in der folgenden Tabelle dargestellt kann dem Haarschädigungsgrad in der Datenbank beispielsweise mindestens eine Produktempfehlung (oder auch eine Styling- oder sonstige Haarbehandlungsempfehlung) zugeordnet sein.

| | |
|---|---|
| Haarschädigungsgrad | Produktempfehlung |
| sehr gering | Produkt mit sehr geringem Pfleganteil |
| gering | Produkt mit geringem Pflegeanteil |
| mittel | Produkt mit mittlerem Pflegeanteil |
| stark | Produkt mit hohem Pflegeanteil |
| sehr stark | Produkt mit sehr hohem Pflegeanteil |

Die erfassten (gemessenen) Sensorwerte bezüglich des Haarzustandsparameters können, wie oben beschrieben, entweder direkt in der Haarbehandlungsvorrichtung 100 ausgewertet werden, z.B. mittels der elektronischen Schaltkreisvorrichtung 104, oder indirekt ausgewertet werden, indem sie an eine externe Datenverarbeitungsvorrichtung 226 übertragen werden, um dort ausgewertet zu werden, z.B. wie oben beschrieben. Dabei können die Sensordaten oder Teile der Sensordaten in verschiedenen Ausführungsbeispielen ausgewertet werden mittels eines Vergleichs mit (z.B. empirisch gewonnenen) Datenbankeinträgen.

In verschiedenen Ausführungsbeispielen kann ein ermittelter Haarzustand (z.B. Haarschädigungsgrad) einbezogen werden bei einem Ermitteln einer Empfehlung, z.B. einer Produkt- oder Behandlungsempfehlung.

In verschiedenen Ausführungsbeispielen kann das ermittelte Haarbehandlungsmittel, z.B. Haarpflegemittel, mittels der Haarbehandlungsvorrichtung oder des Haarbehandlungssystems auf das Haar aufgebracht werden, wobei Steuerungs- bzw. Regelungsparameter zum Dosieren des Haarbehandlungsmittels mittels der elektronischen Schaltkreisvorrichtung 104, der elektronischen Schaltkreisvorrichtung 1040 und/oder der externen Datenverarbeitungsvorrichtung 226 bereitgestellt sein bzw. werden können.

FIG. 3 zeigt eine schematische Darstellung einer Anwendung einer Haarbehandlungsvorrichtung 100 gemäß verschiedenen Ausführungsbeispielen.

Wie in FIG. 3 dargestellt ist, kann die Haarbehandlungsvorrichtung 100 auf für die Haarbehandlungsvorrichtung übliche Weise zur Haarbehandlung genutzt werden. Dargestellt ist ein Glätteisen, in welches die Haare 220H strähnenweise eingeklemmt werden und welches typischerweise in Pfeilrichtung, d.h. vom Haaransatz in Richtung zu den Haarspitzen bewegt wird. Sinngemäß wird beispielsweise bei Verwendung eines Lockenstabs als Haarbehandlungsvorrichtung das Haar strähnenweise auf die Haarbehandlungsvorrichtung 100 aufgewickelt, bei Verwendung eines Kamms als Haarbehandlungsvorrichtung das Haar strähnenweise durchgekämmt, usw.

Für die oben beschriebenen Ermittlungen kann in verschiedenen Ausführungsbeispielen eine Programmierung, z.B. eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum kosmetischen Behandeln von Haaren eines Nutzers gemäß verschiedenen Ausführungsbeispielen ein Smartphone, ein Tablet oder ähnliches genutzt wird, kann die Software als eine App bereitgestellt sein.

In verschiedenen Ausführungsbeispielen kann die in der Haarbehandlungsvorrichtung integrierte Schaltkreisvorrichtung und/oder eine externe Datenverarbeitungsvorrichtung, z.B. ein Smartphone, ein Tablet, ein Laptop, ein Smart Mirror, eine Smart Watch, ein iPad, oder ähnliches geeignet sein, um bei einem Ausführen des Verfahrens zum Bereitstellen einer Haarzustandsinformation, z.B. bei Ermittlungsvorgängen, z.B. mittels Vergleichens mit einer Datenbank/Referenzwerten o.ä., verwendet zu werden. In verschiedenen Ausführungsbeispielen braucht die Programmierung/Software nicht auf dem Smartphone, dem Tablet, dem Laptop usw. bereitgestellt zu werden. Es kann beispielsweise ausreichend sein, wenn die in die Haarbehandlungsvorrichtung integrierte Schaltkreisvorrichtung und/oder das Smartphone o.ä. durch das Internet, mittels WLAN oder auf andere gängige Weise mit einer (z.B. einer weiteren) externen Datenverarbeitungsvorrichtung, z.B. einem Computer, beispielsweise einer Cloud, verbunden ist. In einem solchen Fall können die Berechnungen beispielsweise mittels der (weiteren) externen Datenverarbeitungsvorrichtung, z.B. mittels des Computers, ausgeführt werden, und das Ergebnis kann dem Smartphone/Tablet o.ä. und/oder der internen Schaltkreisvorrichtung bereitgestellt werden.

## Patentansprüche

1. Haarbehandlungsvorrichtung (100), aufweisend:
einen Vorrichtungskörper (100K);
mindestens einen im oder am Vorrichtungskörper (100K) angeordneten Sensor (106) zum Erfassen eines Haarzustandsparameters;
mindestens einen im oder am Vorrichtungskörper (100K) angeordneten Sensor (106) zum Erfassen von Bewegungen und Ortsveränderungen des Vorrichtungskörpers (100K), und eine im oder am Vorrichtungskörper (100K) angeordnete elektronische Schaltkreisvorrichtung (104),
wobei die elektronische Schaltkreisvorrichtung (104) mit den mindestens zwei Sensoren (106) gekoppelt ist zum Empfangen des erfassten Haarzustandsparameters und zum Empfangen der erfassten Bewegungen und Ortsveränderungen,
wobei die elektronische Schaltkreisvorrichtung (104) ferner eingerichtet ist, basierend auf dem empfangenen erfassten Haarzustandsparameter mindestens einen Haar-Behandlungsparameter zu steuern und mindestens ein Haar-Behandlungsmittel zu dosieren und optional
eine Haarbehandlungsempfehlung bereitzustellen und wobei die elektronische Schaltkreisvorrichtung (104) ferner eingerichtet ist, basierend auf den empfangenen Bewegungen und Ortsveränderungen eine räumliche Lage des Vorrichtungskörpers (100K) und/oder eine Geschwindigkeit des Vorrichtungskörpers (100K) zu ermitteln.

2. Haarbehandlungsvorrichtung (100) gemäß Anspruch 1,
wobei die Haarbehandlungsvorrichtung (100) ein Glätteisen aufweist.

3. Haarbehandlungsvorrichtung (100) gemäß Anspruch 1 oder 2,
wobei die elektronische Schaltkreisvorrichtung (104) ermittelt, ob die ermittelte Geschwindigkeit des Vorrichtungskörpers (100K) ein vorgegebenes Kriterium gemäß einer vorgesehenen Geschwindigkeit zum Anwenden der Haarbehandlungsvorrichtung (100) erfüllt.

4. Haarbehandlungsvorrichtung (100) gemäß Anspruch 3,
wobei die elektronische Schaltkreisvorrichtung (104) eingerichtet ist, in Abhängigkeit davon, ob die ermittelte Geschwindigkeit das vorgegebene Kriterium erfüllt ist, ein Signal abzugeben.

5. Haarbehandlungsvorrichtung (100) gemäß Anspruch 4,
wobei das Signal ein akustisches Signal, ein optisches Signal und/oder haptisches Signal umfasst.

6. Haarbehandlungsvorrichtung (100) gemäß Anspruch 4 oder 5,
wobei die Abgabe des Signals während der Anwendung der Haarbehandlungsvorrichtung (100) erfolgt.

7. Haarbehandlungsvorrichtung (100) gemäß einem der Ansprüche 1 bis 6,
wobei die mindestens zwei Sensoren (106) im Vorrichtungskörper (100K) versiegelt angeordnet ist.

8. Haarbehandlungsvorrichtung (100) gemäß einem der Ansprüche 1 bis 7,
wobei die elektronische Schaltkreisvorrichtung (104) eine kabellose Datenaustauschvorrichtung (104a) aufweist.

9. Haarbehandlungsvorrichtung (100) gemäß einem der Ansprüche 1 bis 8,
wobei die elektronische Schaltkreisvorrichtung (104) eingerichtet ist, basierend auf der ermittelten räumlichen Lage des Vorrichtungskörpers (100K) zu ermitteln, welche Haarbereiche bereits mit der Haarbehandlungsvorrichtung (100) behandelt wurden.

10. Haarbehandlungsvorrichtung (100) gemäß einem der Ansprüche 1 bis 9,
wobei der Sensor (106) zum Erfassen von Bewegungen und Ortsveränderungen des Vorrichtungskörpers (100K) ausgewählt ist aus der Gruppe bestehend aus Magnetfeldsensoren, Gyroskopen, Beschleunigungssensoren und mechanisch arbeitenden Wegmesssensoren.

## Claims

1. A hair treatment device (100), comprising:
a device body (100K);
at least one sensor (106) arranged in or on the device body (100K) for detecting a hair state parameter;
at least one sensor (106) arranged in or on the device body (100K) for detecting movements and changes in position of the device body (100K), and an electronic circuit device (104) arranged in or on the device body (100K),
wherein the electronic circuit device (104) is coupled to the at least two sensors (106) in order to receive the detected hair state parameter and to receive the detected movements and changes in position,
wherein the electronic circuit device (104) is further configured to control, on the basis of the received detected hair state parameter, at least one hair treatment parameter and to meter at least one hair treatment agent and optionally provide a hair treatment recommendation, and wherein the electronic circuit device (104) is further configured to determine, on the basis of the received movements and changes in position, a spatial location of the device body (100K) and/or a speed of the device body (100K).

2. The hair treatment device (100) according to claim 1,
wherein the hair treatment device (100) comprises a straightening iron.

3. The hair treatment device (100) according to claim 1 or 2,
wherein the electronic circuit device (104) determines whether the determined speed of the device body (100K) satisfies a predetermined criterion according to a provided speed for applying the hair treatment device (100).

4. The hair treatment device (100) according to claim 3,
wherein the electronic circuit device (104) is configured to emit a signal depending on whether the determined speed satisfies the predetermined criterion.

5. The hair treatment device (100) according to claim 4,
wherein the signal comprises an acoustic signal, an optical signal and/or a haptic signal.

6. The hair treatment device (100) according to claim 4 or 5,
wherein the signal is emitted during application of the hair treatment device (100).

7. The hair treatment device (100) according to one of claims 1 to 6,
wherein the at least two sensors (106) are arranged so as to be sealed in the device body (100K).

8. The hair treatment device (100) according to one of claims 1 to 7,
wherein the electronic circuit device (104) comprises a wireless data exchange device (104a).

9. The hair treatment device (100) according to one of claims 1 to 8,
wherein the electronic circuit device (104) is configured to determine, on the basis of the determined spatial position of the device body (100K), which hair regions have already been treated with the hair treatment device (100).

10. The hair treatment device (100) according to one of claims 1 to 9,
wherein the sensor (106) for detecting movements and changes in position of the device body (100K) is selected from the group consisting of magnetic field sensors, gyroscopes, acceleration sensors and mechanically operating displacement sensors.

## Revendications

1. Dispositif de traitement des cheveux (100), présentant :
un corps de dispositif (100K) ;
au moins un capteur (106) disposé dans ou sur le corps de dispositif (100K) pour la détection d'un paramètre d'état des cheveux ;
au moins un capteur (106) disposé dans ou sur le corps de dispositif (100K) pour la détection de mouvements et de modifications d'emplacement du corps de dispositif (100K), et un dispositif de circuit électronique (104) disposé dans ou sur le corps de dispositif (100K),
le dispositif de circuit électronique (104) étant couplé aux au moins deux capteurs (106) pour la réception du paramètre d'état des cheveux détecté et pour la réception des mouvements et des modifications d'emplacement détectés,
le dispositif de circuit électronique (104) étant en outre configuré pour, sur la base du paramètre d'état des cheveux détecté reçu, commander au moins un paramètre de traitement des cheveux et doser au moins un agent de traitement des cheveux et éventuellement mettre à disposition une recommandation de traitement des cheveux et le dispositif de circuit électronique (104) étant en outre configuré pour, sur la base des mouvements et des modifications d'emplacement reçus, déterminer une position spatiale du corps de dispositif (100K) et/ou une vitesse du corps de dispositif (100K).

2. Dispositif de traitement des cheveux (100) selon la revendication 1,
le dispositif de traitement des cheveux (100) présentant un fer à lisser.

3. Dispositif de traitement des cheveux (100) selon la revendication 1 ou 2,
le dispositif de circuit électronique (104) déterminant si la vitesse déterminée du corps de dispositif (100K) satisfait à un critère prédéfini selon une vitesse prévue pour l'utilisation du dispositif de traitement des cheveux (100).

4. Dispositif de traitement des cheveux (100) selon la revendication 3,
le dispositif de circuit électronique (104) étant configuré pour émettre un signal en fonction du fait de savoir si la vitesse déterminée satisfait au critère prédéfini.

5. Dispositif de traitement des cheveux (100) selon la revendication 4,
le signal comprenant un signal acoustique, un signal optique et/ou un signal haptique.

6. Dispositif de traitement des cheveux (100) selon la revendication 4 ou 5,
l'émission du signal ayant lieu pendant l'utilisation du dispositif de traitement des cheveux (100).

7. Dispositif de traitement des cheveux (100) selon l'une des revendications 1 à 6,
les au moins deux capteurs (106) est disposé de manière scellée dans le corps de dispositif (100K).

8. Dispositif de traitement des cheveux (100) selon l'une des revendications 1 à 7,
le dispositif de circuit électronique (104) présentant un dispositif d'échange de données sans fil (104a).

9. Dispositif de traitement des cheveux (100) selon l'une des revendications 1 à 8,
le dispositif de circuit électronique (104) étant configuré pour, sur la base de la position spatiale déterminée du corps de dispositif (100K), déterminer quelles zones des cheveux avaient déjà été traitées par le dispositif de traitement des cheveux (100).

10. Dispositif de traitement des cheveux (100) selon l'une des revendications 1 à 9,
le capteur (106) pour la détection de mouvements et de modifications d'emplacement du corps de dispositif (100K) étant choisi dans le groupe constitué par les capteurs de champ magnétique, les gyroscopes, les capteurs d'accélération et les capteurs de mesure de déplacement fonctionnant de manière mécanique.
